# EUROPEAN PATENT SPECIFICATION

(11) **EP 1 838 840 B1**
(45) Date of publication and mention of the grant of the patent: **09.03.2011**
(21) Application number: 05849967.4
(22) Date of filing: 22.11.2005
(51) Int. Cl.: A61L 27/38, C12N 5/071, A61P 9/00

(54) **USE OF CULTURED THREE-DIMENSIONAL TISSUE FOR TREATING CONGESTIVE HEART FAILURE**
VEWENDUNG VON DREIDIMENSIONALEM KULTIVIERTEM GEWEBE ZUR BEHANDLUNG VON KONGESTIVEM HERZVERSAGEN
UTILISATION DE TISSU TRIDIMENSIONNEL CULTIVE POUR LE TRAITEMENT DE L'INSUFFISANCE CARDIAQUE CONGESTIVE

(30) Priority: 22.11.2004 US 630243 P; 17.06.2005 US 692054 P
(43) Date of publication of application: 03.10.2007
(73) Proprietor: Theregen, Inc., San Francisco CA 94131 (US)
(72) Inventor: SIANI-ROSE, Michael, San Francisco, CA 94131 (US); KELLAR, Robert, S., Flagstaff, AZ 86004 (US); NAUGHTON, Gail, K., San Diego, CA 92101 (US); WILLIAMS, Stuart, K., Tuscon, AZ 85718 (US)
(74) Representative: Chapman, Paul Gilmour
(86) International application number: PCT/US2005/042698
(87) International publication number: WO 2006/055981

(56) References cited:
- WO-A-2004/074457
- US-A- 6 099 832
- US-A1- 2003 003 089
- US-A1- 2003 007 954

## Description

### 2. BACKGROUND

Congestive heart failure (CHF) is a clinical syndrome in which an abnormality of cardiac function causes cardiac output to fall below a level adequate to meet the metabolic demand of peripheral tissues. Congestive heart failure often occurs because other cardiac conditions, such as coronary artery disease lead to myocardial infarction and cardiac muscle death. Additional pathologies that can lead to congestive heart failure include ischemia, cardiomyopathies, myocarditis, valvular dysfunction, metabolic and endocrine abnormalities (e.g., hypothyroidism, alcohol, magnesium deficiency, etc.), pericardial abnormalities, congenital heart defects, heart arrhythmias, and viral infections.

Treatments typically include the use of a number of different pharmaceutical agents, such as angiotensin-converting (ACE) enzyme inhibitors, diuretics, beta blockers, surgical procedures, or both. Although these treatments can improve the symptoms associated with congestive heart failure, they are imperfect. For example, they can not reverse the damage done to the heart. The only permanent treatment for CHF is heart transplant. Consequently, there is a need for additional treatments, especially those that can ameliorate and/or reverse the damage to the heart of a patient diagnosed with CHF.

### 3. SUMMARY

The present invention provides a cultured three-dimensional tissue and uses thereof, as defined in the appended claims. The compositions and uses described herein can be used to treat at least one clinical symptom associated with congestive heart failure. Congestive heart failure is typically caused by a weakening of the heart muscle, leaving it unable to pump enough blood to the peripheral organs. Over time, the weakened heart muscle progressively enlarges until the heart becomes so enlarged that it cannot adequately supply blood. Generally, the use comprises the intended administration of cultured three-dimensional tissues capable of being administered by injection, implantation and/or attachment. For example, in accordance with the present invention, the three-dimensional tissues may be attached using glues, staples, suture, or other means known to those skilled in the art.

In some embodiments, application of three-dimensional tissue(s) is used to strengthen weakened heart muscle such that there is a noticeable increase in pumping efficiency. Changes in the pumping efficiency of the heart can be measured using various methods, including echocardiograms and nuclear scans.

In some embodiments, application of three-dimensional tissue(s) reduces the size of the heart by facilitating the remodeling of the treated heart tissue, for example, by promoting endothelialization, tissue growth, vascularization and/or angiogenesis in the treated heart tissue. Various methods can be used to visualize changes in the heart tissue following application of three-dimensional tissue(s), including X-ray and laser Doppler imaging.

In other embodiments, application of three-dimensional tissue(s) improves the contractility of the heart. Improvements in the contractility of the heart can be measured by a number of different methods, including radionuclide ventriculography or multiple gated acquisition scanning to determine how much blood the heart pumps with each beat

Various cell types can be used to forum the three-dimensional tissues. The three-dimensional tissues can be formed from one cell type, or various combinations of cell types can be used to form the three-dimensional tissues. Examples of suitable cell types include, but are not limited to stromal cells, fibroblasts, cardiac muscle cells, stem cells, and/or other cells of tissue specific origin. Genetically engineered cells can also be used to form the three-dimensional tissues described herein.

In some embodiments, the three-dimensional tissues are used as a source of, or to deliver, various growth factors produced by the three-dimensional tissues, including VEGF and Wnt proteins.

The amount of cultured three-dimensional tissue(s) administered, can vary, depending in part, on the severity of the clinical symptoms being manifested in an individual being treated for congestive heart failure. For example, one, two, three or more cohesive pieces of cultured three-dimensional tissue can be used to increase the area of treatable heart tissue, increase the concentration of the various growth factors and/or Wnt proteins present, and/or increase the number of viable cells being delivered by the compositions and methods described herein.

In some embodiments, the compositions and uses described herein are combined with conventional treatments, such as the administration of various pharmaceutical agents and surgical procedures to treat individuals diagnosed with congestive heart failure. For example, in some embodiments, application of the three-dimensional tissue is used with other options to treat severe heart failure, including heart pumps, biventricular cardiac pacemakers and cardiac wrap surgery.

### 4. BRIEF DESCRIPTION OF THE FIGURES

FIG. 1 depicts histological evidence of new microvessel formation in canine dog hearts contacted with Anginera™ according to some of the embodiments described herein;

FIGS. 2A and 2B depict end diastolic volume index (EDVI) parameters during the 30 day ameroid period according to some of the embodiments described herein;

FIG. 3 depicts the cardiac output in the four treatment groups 30 days after placement of Anginera™ according to some of the embodiments described herein;

FIG. 4 depicts the cardiac output in the four treatment groups 90 days after placement of Anginera™ according to some of the embodiments described herein;

FIG. 5 depicts the left ventricular (LV) ejection fraction in the four treatment groups 30 days after placement of Anginera™;

FIG. 6 depicts the LV ejection fraction in the four treatment groups 90 days after placement of Anginera™ according to some of the embodiments described herein;

FIG. 7 depicts the LV end diastolic volume in the four treatment groups at stress 30 and 90 days after placement of Anginera accordingly to some of the embodiments described herein;

FIG. 8 depicts the LV end diastolic volume in the four treatment groups at rest 30 and 90 days after placement of Anginera™ according to some of the embodiments described herein;

FIG. 9 depicts the LV systolic volume in the four treatment groups at rest/stress 30 days after placement of Anginera™;

FIG. 10 depicts the LV systolic volume in the four treatment groups at rest/stress 90 days after placement of Anginera™ according to some of the embodiments described herein;

FIG. 11 depicts systolic wall thickening in early ischemia in the four treatment groups 30 days after placement of Anginera™ according to some of the embodiments, described herein;

FIG. 12 depicts systolic wall thickening in chronic ischemia in the four treatment groups 30 days after placement of Anginera™ according to some of the embodiments described herein;

FIGS. 13A and 13B depict the injection of cultured beads from a 24 gauge needle equipped Hamilton Syringe into ischemic hindlimb tissue according to some of the embodiments described herein;

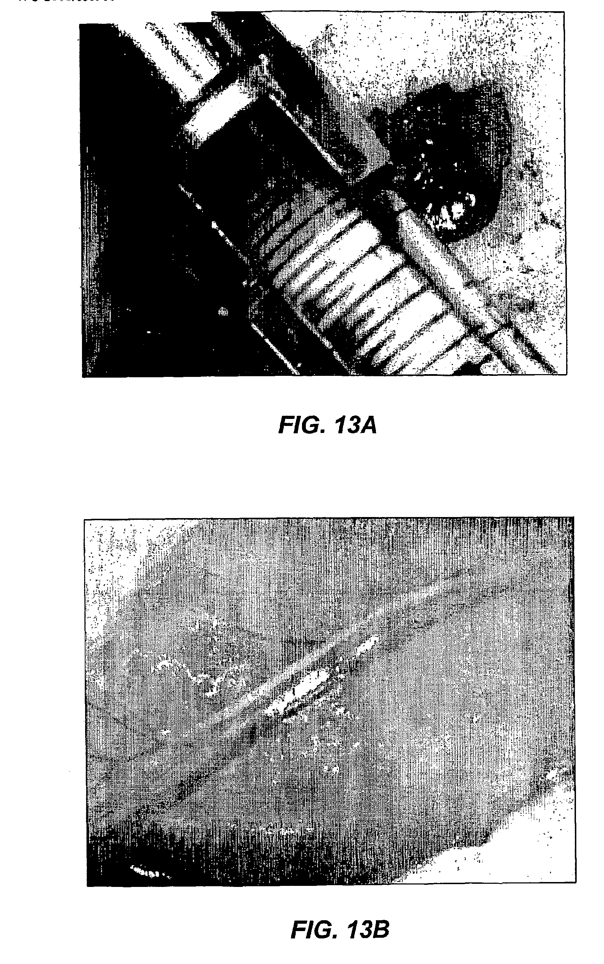

FIGS 13A and 13B are photographs of ischemia only treated animals two weeks after inducing ischemia according to some of the embodiments described herein;

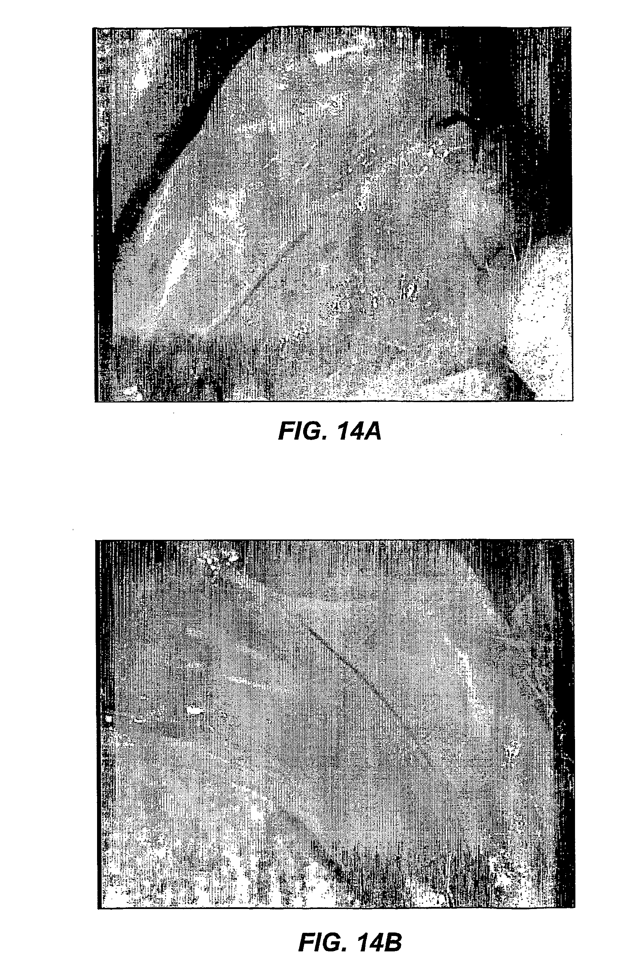

FIGS. 14A and 14B are photographs of two week explants of three dimensional tissues formed on microparticles, showing evidence of limited new microvessel formation (black arrows) in ischemic limbs treated with SMC grown on Alkermes® beads according to some of the embodiments describe herein; and

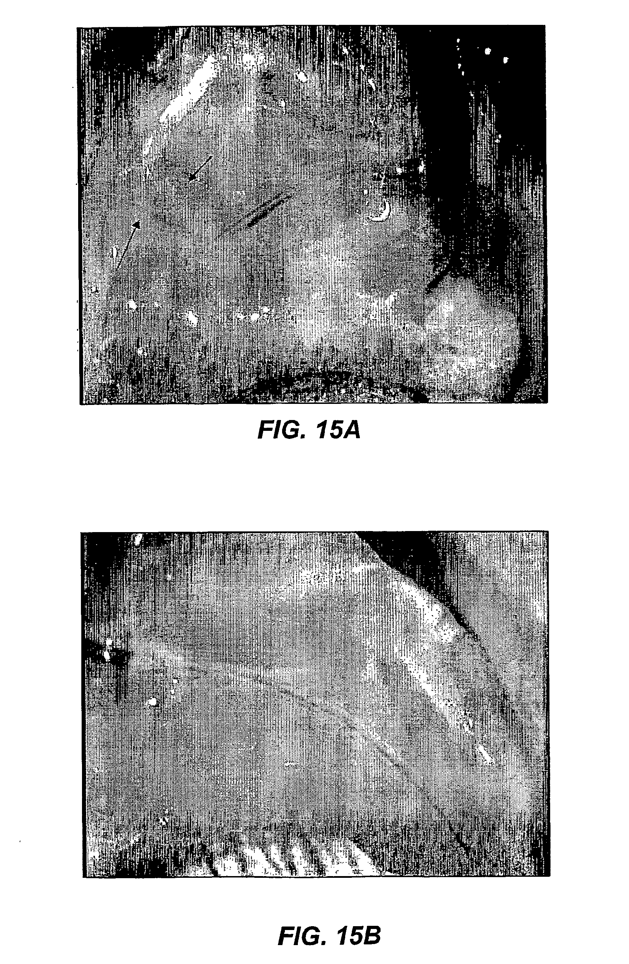

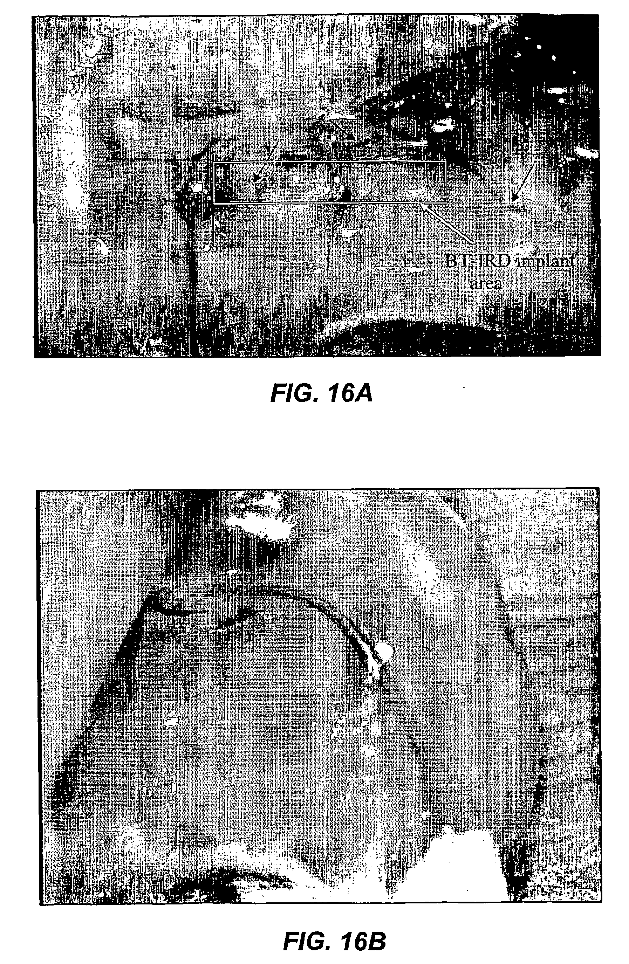

FIGS. 15A and 15B show new microvessel formation (black arrows) surrounding braided threads after 14 days of implantation according to some of the embodiments described herein.

### 5. DETAILED DESCRIPTION

Disclosed herein are compositions for treating a patient suffering from congestive heart failure, comprising contacting a region of the patient's heart with an amount of a cultured three-dimensional tissue that comprises living cells effective to treat at least one clinical symptom associated with congestive heart failure.

Clinically, congestive heart failure involves circulatory congestion caused by heart disorders that are primarily characterized by abnormalities of left and/or right ventricular function and neurohormonal regulation. Congestive heart failure occurs when these abnormalities cause the heart to fail to pump blood at a rate required by metabolizing tissues. Congestive heart failure can involve the left side, right side or both sides of the heart. Typically heart failure begins with the left side, specifically the left ventricle. In left ventricular failure, fluid accumulation occurs in the lungs and is manifest by pulmonary edema. Left heart failure is more common and important because of its relative size and the physiologic function of providing systemic circulation to critical organs. Three medical conditions can be associated with heart failure that begins in the left ventricle: systolic heart failure, diastolic heart failure, or a combination of both.

Right sided heart failure can occur independently or be a consequence of left ventricular heart failure. In the case of right ventricular failure, the consequence is systemic edema often initially manifest by symptomatic dependent edema at peripheral sites (*e.g*. ankles).

### 5.2 Three Dimensional Tissue and Scaffolds

In various embodiments, the three-dimensional tissue capable of promoting healing of heart tissue in individuals diagnosed with congestive heart failure can be obtained from various types of cells as discussed in more detail below. The three-dimensional tissue can be obtained commercially or generated *de novo* using the procedures described in U.S. Patent Nos. 6,372,494; 6,291,240; 6121,042; 6,022,743; 5,962,325; 5,858,721; 5,830,708; 5,785,964; 5,624,840; 5,512,475; 5,510,254; 5,478,739; 5,443,950; and 5,266,480.

In some embodiments, the cultured three-dimensional tissue is obtained commercially from Smith & Nephew, London, United Kingdom. In particular, the product referred to as Dermagraft™, also referred to herein as Anginera™, can be obtained from Smith & Nephew.

Generally, the cultured cells are supported by a scaffold, also referred to herein as a three-dimensional framework, composed of a biocompatible, non-living material. The scaffold can be of any material and/or shape that: (a) allows cells to attach to it (or can be modified to allow cells to attach to it); and (b) allows cells to grow in more than one layer (*i.e*., form a three dimensional tissue).

In some embodiments, the biocompatible material is formed into a three-dimensional scaffold comprising interstitial spaces for attachment and growth of cells into a three dimensional tissue. The openings and/or interstitial spaces of the scaffold are of an appropriate size to allow the cells to stretch across the openings or spaces. Maintaining actively growing cells that are stretched across the scaffold appears to enhance production of the repertoire of growth factors responsible for the activities described herein. If the openings are too small, the cells may rapidly achieve confluence but be unable to easily exit from the mesh. These trapped cells may exhibit contact inhibition and cease production of the growth factors described herein. If the openings are too large, the cells may be unable to stretch across the opening; which may decrease production of the growth factors described herein. When using a mesh type of scaffold, as exemplified herein, it has been found that openings at least about 140 µm, at least about 150 µm, at least about 160 µm, at least about 175 µm, at least about 185 µm, at least about 200 µm, at least about 210 µm, and at least about 220 µm work satisfactorily. However, depending upon the three-dimensional structure and intricacy of the scaffold, other sizes can work equally well. In fact, any shape or structure that allows the cells to stretch, replicate, and grow for a suitable length of time to elaborate the growth factors described herein can be used.

In some embodiments, the three dimensional scaffold is formed from polymers or threads braided, woven, knitted or otherwise arranged to form a scaffold, such as a mesh or fabric. The materials can be formed by casting the material or fabrication into a foam, matrix, or sponge-like scaffold. In other embodiments, the three dimensional scaffold is in the form of matted fibers made by pressing polymers or other fibers together to generate a material with interstitial spaces. The three dimensional scaffold can take any form or geometry for the growth of cells in culture as long as the resulting tissue expresses one or more of the tissue healing activities described herein. Descriptions of cell cultures using a three dimensional scaffold are described in U.S. Patent Nos. 6,372,494; 6,291,240; 6121,042; 6,022,743; 5,962,325; 5,858,721; 5,830,708; 5,785,964; 5,624,840; 5,512,475; 5,510,254; 5,478,739; 5,443,950; and 5,266,480.

A number of different materials can be used to form the scaffold. These materials can be non-polymeric and/or polymeric materials. Polymers when used can be any type of block polymers, co-block polymers (*e.g*., di, tri, etc.), linear or branched polymers, crosslinked or non-crosslinked. Non-limiting examples of materials for use as scaffolds or frameworks include, among others, glass fiber, polyethylene, polypropylene, polyamides (*e.g*., nylon), polyesters (*e.g*., Dacron), polystyrenes, polyacrylates, polyvinyl compounds (*e.g*., polyvinylchloride; PVC), polycarbonates, polytetrafluorethylenes (PTFE; TEFLON), expanded PTFE (ePTFE), thermanox (TPX), nitrocellulose, polysaacharides (*e.g*., celluloses, chitosan, agarose), polypeptides (*e.g*., silk, gelatin, collagen), polyglycolic acid (PGA), and dextran.

In some embodiments, the scaffold is comprised of materials that degrade over time under the conditions of use. As used herein, a "degradable material" refers to a material that degrades or decomposes. In some embodiments, the degradable material is biodegradable, *i.e*., degrades through action of biological agents, either directly or indirectly. Non-limiting examples of degradable materials include, among others, poly(lactic-co-glycolic acid) (*i.e*., PLGA), trimethylene carbonate (TMC), co-polymers of TMC, PGA, and/or PLA, polyethylene terephtalate (PET), polycaprolactone, catgut suture material, collagen (*e.g*., equine collagen foam), polylactic acid (PLA), fibronectin matrix, or hyaluronic acid.

In other embodiments, the three dimensional scaffold is a combination of degradeable and non-degradeable materials. The non-degradable material provides stability to the scaffold during culturing, while the degradeable material allows interstitial spaces to form sufficient for formation of three-dimensional tissues that produce factors useful for treating congestive heart failure. The degradable material can be coated onto the non-degradable material or woven, braided or formed into a mesh. Various combinations of degradable and non-degradable materials can be used. An exemplary combination is poly(ethylene therephtalate) (PET) fabrics coated with a thin degradable polymer film (poly[D-L-lactic-co-glycolic acid] PLGA).

In various embodiments, the scaffold material is pre-treated prior to inoculation with cells to enhance cell attachment to the scaffold. For example, prior to inoculation with cells, nylon screens are treated with 0.1 M acetic acid, and incubated in polylysine, fetal bovine serum, and/or collagen to coat the nylon. In some embodiments, polystyrene can be analogously treated using sulfuric acid. In other embodiments, the growth of cells in the presence of the three-dimensional scaffold is further enhanced by adding to the scaffold, or coating with proteins (e.g., collagens, elastin fibers, reticular fibers), glycoproteins, glycosaminoglycans (e.g., heparan sulfate, chondroitin-4-sulfate, chondroitin-6-sulfate, dermatan sulfate, keratan sulfate, etc.), fibronectins, a cellular matrix, glycopolymer (poly[N-p-vinylbenzyl-D-lactoamide], PVLA) and/or other materials in order to improve cell attachment.

In other embodiments, the scaffold comprises particles so dimensioned such that cells cultured in the presence of the particles elaborate factors that promote healing of weakened heart tissue in individuals diagnosed with congestive heart failure. In some embodiments, the particles comprise microparticles, or other suitable particles, such as microcapsules and nanoparticles, which can be degradable or non-degradable (see, *e.g*., *"*Microencapsulates : Methods and Industrial Applications, " in Drugs and Pharmaceutical Sciences, 1996, Vol 73, Benita, S. ed, Marcel Dekker Inc., New York). Generally, the microparticles have a particle size range of at least about 1 µm, at least about 10 µm, at least about 25 µm, at least about 50 µm, at least about 100 µm, at least about 200 µm, at least about 300 µm, at least about 400 µm, at least about 500 µm, at least about 600 µm, at least about 700 µm, at least about 800 µm, at least about 900 µm, at least about 1000 µm. Nanoparticles have a particle size range of at least about 10 nm, at least about 25 µm, at least about 50 nm, at least about 100 nm, at least about 200 nm, at least about 300 nm, at least about 400 nm, at least about 500 nm, at least about 600 nm, at least about 700 nm, at least about 800 nm, at least about 900 nm, at least about 1000 nm. The microparticles can be porous or nonpororus. Various microparticle formulations can be used for preparing the three-dimensional scaffold, including microparticles made from degradable or non-degradable materials used to form the mesh or woven polymers described above.

Exemplary non-degradable microparticles include, but are not limited to, polysulfones, poly (acrylonitrile-co-vinyl chloride), ethylene-vinyl acetate, hydroxyethylmethacrylate-methyl-methacrylate copolymers. Degradable microparticles include those made from fibrin, casein, serum albumin, collagen, gelatin, lecithin, chitosan, alginate or poly-amino acids such as poly-lysine. Degradable synthetic polymers polymers such as polylactide (PLA), polyglycolide (PGA), poly (lactide-co-glycolide) (PLGA), poly (caprolactone), polydioxanone trimethylene carbonate, polyhybroxyalkonates (e. g., poly (y-hydroxybutyrate)), poly (Y-ethyl glutamate), poly (DTH iminocarbony (bisphenol A iminocarbonate), poly (ortho ester), and polycyanoacrylate.

Hydrogels can also be used to provide three-dimensional scaffolds. Generally, hydrogels are crosslinked, hydrophilic polymer networks. Non-limiting examples of polymers useful in hydrogel compositions include, among others, those formed from polymers of poly (lactide- co-glycolide), poly (N-isopropylacrylamide, poly (methacrylic acid-y-polyethylene glycol), polyacrylic acid and poly (oxypropylene-co-oxyethylene) glycol, and natural compounds such as chrondroitan sulfate, chitosan, gelatin, fibrinogen, or mixtures of synthetic and natural polymers, for example chitosan-poly (ethylene oxide). The polymers can be crosslinked reversibly or irreversibly to form gels sufficient for cells to attach and form a three-dimensional tissue.

Various methods for making microparticles are well known in the art, including solvent removal process (see, *e.g*., US Patent No. 4,389,330); emulsification and evaporation (Maysinger et al., 1996, Exp. Neuro. 141: 47-56; Jeffrey et al., 1993, Pharm. Res. 10: 362-68), spray drying, and extrusion methods. Exemplary microparticles for preparing three dimensional scaffolds are described in US Publication No. 2003/0211083 and US Patent Nos. 5,271,961; 5,413,797; 5,650,173; 5,654,008; 5,656,297; 5,114,855; 6,425,918; and 6,482,231, and U.S. application no. 11/216,574, entitled "Cultured Three Dimensional Tissues and Uses Thereof," filed August 30, 2005.

It is to be understood that other materials in various geometric forms, other than those described above, can be used to generate a three-dimensional tissue with the tissue healing characteristics described herein, and thus, the materials are not limited to the specific embodiments disclosed herein.

### 5.3 Cells and Culture Conditions

In some embodiments, the cultured three dimensional-tissues are made by inoculating the biocompatible materials comprising the three-dimensional scaffold with the appropriate cells and growing the cells under suitable conditions to promote production of a cultured three-dimensional tissue with one or more tissue healing properties. Cells can be obtained directly from a donor, from cell cultures made from a donor, or from established cell culture lines. In some instances, cells can be obtained in quantity from any appropriate cadaver organ or fetal source. In some embodiments, cells of the same species preferably matched at one or more MHC loci, are obtained by biopsy, either from the subject or a close relative, which are then grown to confluence in culture using standard conditions and used as needed. The characterization of the donor cells is made in reference to the subject being treated with the three-dimensional tissue.

In some embodiments, the cells are autologous, *i*.*e*., the cells are derived from the recipient. Because the three-dimensional tissue is derived from the recipient's own cells, the possibility of an immunological reaction that neutralizes the activity of the three-dimensional tissue is reduced. In these embodiments, cells are typically cultured to obtain a sufficient number to produce the three-dimensional tissue.

In other embodiments, the cells are obtained from a donor who is not the intended recipient of the three-dimensional tissue. In some of these embodiments, the cells are syngeneic, derived from a donor who is genetically identical at all MHC loci. In other embodiments, the cells are allogeneic, derived from a donor differing in at least one MHC locus from the intended recipient. When the cells are allogeneic, the cells can be from a single donor or comprise a mixture of cells from different donors who themselves are allogeneic to each other. In further embodiments, the cells comprise xenogenic, i. e., they are derived from a species that is different from the intended recipient.

In various embodiments herein, the cells inoculated onto the scaffold can be stromal cells comprising fibroblasts, with or without other cells, as further described below. In some embodiments, the cells are stromal cells, that are typically derived from connective tissue, including, but not limited to: (1) bone; (2) loose connective tissue, including collagen and elastin; (3) the fibrous connective tissue that forms ligaments and tendons, (4) cartilage; (5) the extracellular matrix of blood; (6) adipose tissue, which comprises adipocytes; and, (7) fibroblasts.

Stromal cells can be derived from various tissues or organs, such as skin, heart, blood vessels, skeletal muscle, liver, pancreas, brain, foreskin, which can be obtained by biopsy (where appropriate) or autopsy.

The fibroblasts can be from a fetal, neonatal, adult origin, or a combination thereof. In some embodiments, the stromal cells comprise fetal fibroblasts. As used herein a "fetal fibroblast" refers to fibroblasts derived from fetal sources. As used herein a "neonatal fibroblast" refers to fibroblasts derived from newborn sources. Under appropriate conditions, fibroblasts can give rise to other cells, such as bone cells, fat cells, and smooth muscle cells and other cells of mesodermal origin. In some embodiments, the fibroblasts comprise dermal fibroblasts. As used herein, dermal fibroblasts refers to fibroblasts derived from skin. Normal human dermal fibroblasts can be isolated from neonatal foreskin. These cells are typically cryopreserved at the end of the primary culture.

In other embodiments, the three-dimensional tissue is made using stem and/or progenitor cells, either alone, or in combination with any of the cell types discussed herein. The term "stem cell" includes, but is not limited to, non-human embryonic stem cells, hematopoietic stem cells, neuronal stem cells, and mesenchymal stem cells.

In some embodiments, a "specific" three-dimensional tissue is prepared by inoculating the three-dimensional scaffold with cells derived from a particular organ, *i.e*., skin, heart, and/or from a particular individual who is later to receive the cells and/or tissues grown in accordance with the methods described herein.

As discussed above, additional cells can be present in the culture with the stromal cells. Additional cell types include, but are not limited to, smooth muscle cells, cardiac muscle cells, endothelial cells and/or skeletal muscle cells. In some embodiments, fibroblasts, along with one or more other cell types, are inoculated onto the three-dimensional scaffold. Examples of other cell types include, but are not limited to, cells found in loose connective tissue, endothelial cells, pericytes, macrophages, monocytes, adipocytes, skeletal muscle cells, smooth muscle cells, and cardiac muscle cells. These other cell types can readily be derived from appropriate tissues or organs such as skin, heart, and blood vessels, using methods known in the art such as those discussed below. In other embodiments, one or more other cell types, excluding fibroblasts, are inoculated onto the three-dimensional scaffold. In still other embodiments, the three-dimensional scaffolds are inoculated only with fibroblasts cells.

Cells useful in the uses and compositions described herein can be isolated by disaggregating an appropriate organ or tissue. This can be accomplished using techniques known to those skilled in the art. For example, the tissue or organ can be disaggregated mechanically and/or treated with digestive enzymes and/or chelating agents that weaken the connections between neighboring cells making it possible to disperse the tissue into a suspension of individual cells without appreciable cell breakage. Enzymatic dissociation can be accomplished by mincing the tissue and treating the minced tissue with any of a number of digestive enzymes, either alone or in combination. These include, but are not limited to, trypsin, chymotrypsin, collagenase, elastase, and/or hyaluronidase, DNase, pronase, and dispase. Mechanical disruption can be accomplished by a number of methods including, but not limited to, the use of grinders, blenders, sieves, homogenizers, pressure cells, or insonators to name but a few. For a review of tissue disaggregation techniques, see Freshney, Culture of Animal Cells. A Manual of Basic Technique, 2d Ed., A.R. Liss, Inc., New York, 1987, Ch. 9, pp. 107-126.

Once the tissue has been reduced to a suspension of individual cells, the suspension can be fractionated into subpopulations from which the fibroblasts and/or other stromal cells and/or other cell types can be obtained. This can be accomplished using standard techniques for cell separation including, but not limited to, cloning and selection of specific cell types, selective destruction of unwanted cells (negative selection), separation based upon differential cell agglutinability in the mixed population, freeze-thaw procedures, deferential adherence properties of the cells in the mixed population, filtration, conventional and zonal centrifugation, centrifugal elutriation (counter-streaming centrifugation), unit gravity separation, countercurrent distribution, electrophoresis and fluorescence-activated cell sorting. For a review of clonal selection and cell separation techniques, see Freshney, Culture ofAnimal Cells. A Manual of Basic Techniques, 2d Ed., A.R. Liss, Inc., New York, 1987, Ch. 11 and 12, pp. 137-168.

Cells suitable for use in the uses and compositions described herein can be isolated, for example, as follows: fresh tissue samples are thoroughly washed and minced in Hanks balanced salt solution (HBSS) in order to remove serum. The minced tissue is incubated from 1-12 hours in a freshly prepared solution of a dissociating enzyme such as trypsin. After such incubation, the dissociated cells are suspended, pelleted by centrifugation and plated onto culture dishes. As stromal cells attach before other cells, appropriate stromal cells can be selectively isolated and grown. The isolated stromal cells can be grown to confluency, lifted from the confluent culture and inoculated onto the three-dimensional scaffold (U.S. Patent No. 4,963,489; Naughton et al., 1987, J. Med. 18(3&4):219-250). Inoculation of the three-dimensional scaffold with a high concentration of cells, *e*.*g*., approximately 1 x 10⁶ to 5 x 10⁷ stromal cells/ml, can result in the establishment of a three-dimensional tissue in shorter periods of time.

In other embodiments, an engineered three-dimensional tissue prepared on a three-dimensional scaffold includes tissue-specific cells and produces naturally secreted growth factors and Wnt proteins that stimulate proliferation or differentiation of stem or progenitor cells into specific cell types or tissues. Moreover, the engineered three-dimensional tissue can be engineered to include stem and/or progenitor cells. Examples of stem and/or progenitor cells that can be stimulated by and/or included within the engineered three-dimensional tissue, include, but are not limited to, stromal cells, parenchymal cells, mesenchymal stem cells, liver reserve cells, neural stem cells, pancreatic stem cells and/or embryonic stem cells.

After inoculation of the scaffold with desired cell type(s), the scaffold can be incubated in an appropriate nutrient medium that supports the growth of the cells into a three-dimensional tissue. Many commercially available media, such as Dulbecco's Modified Eagles Medium (DMEM), RPMI 1640, Fisher's, and Iscove's, McCoy's, are suitable for use. The medium can be supplemented with additional salts, carbon sources, amino acids, serum and serum components, vitamins, minerals, reducing agents, buffering agents, lipids, nucleosides, antibiotics, attachment factors, and growth factors. Formulations for different types of culture media are described in reference works available to the skilled artisan *(e.g.,* Methods for Preparation of Media, Supplements and Substrates for Serum Free Animal Cell Cultures, Alan R. Liss, New York (1984); Tissue Culture: Laboratory Procedures, John Wiley & Sons, Chichester, England (1996); Culture of Animal Cells, A Manual of Basic Techniques, 4th Ed., Wiley-Liss (2000)). Typically, the three-dimensional tissue is suspended in the medium during the incubation period in order to enhance tissue healing activity(ies), secretion of growth factors and/or Wnt proteins. In some embodiments, the culture can be "fed" periodically to remove spent media, depopulate released cells, and add fresh medium. During the incubation period, the cultured cells grow linearly along and envelop the filaments of the three-dimensional scaffold before beginning to grow into the openings of the scaffold.

Different proportions of various types of collagen deposited on the scaffold can affect the growth of the cells that come in contact with the three-dimensional tissue. The proportions of extracellular matrix (ECM) proteins deposited can be manipulated or enhanced by selecting fibroblasts which elaborate the appropriate collagen type. This can be accomplished using monoclonal antibodies of an appropriate isotype or subclass that is capable of activating complement, and which define particular collagen types. These antibodies and complement can be used to negatively select the fibroblasts which express the desired collagen type. Alternatively, the cells used to inoculate the framework can be a mixture of cells that synthesize the desired collagen types. The distribution and origin of different collagen types is shown in Table 1.

| **Table 1** | | |
|---|---|---|
| **Collagen Type** | **Principle Tissue Distribution** | **Cells of Origin** |
| I | Loose and dense ordinary connective tissue; collagen fibers | Fibroblasts and reticular cells; smooth muscle cells |
| | Fibrocartilage | |
| | Bone | Osteoblast |
| | Dentin | Odontoblasts |
| II | Hyaline and elastic cartilage | Chondrocytes |
| | Vitreous body of the eye | Retinal cells |
| III | Loose connective tissue; reticular fibers | Fibroblasts and reticular cells |
| | Papillary layer of dermis | |
| | Blood vessels | Smooth muscle cells; endothelial cells |
| IV | Basement membranes | Epithelial and endothelial cells |
| | Lens capsule of the eye | Lens fiber |
| V | Fetal membranes; placenta | Fibroblasts |
| | Basement membranes | |
| | Bone | |
| | Smooth muscle | Smooth muscle cells |
| VI | Connective tissue | Fibroblasts |
| VII | Epithelial basement membranes; anchoring fibrils | Fibroblasts; keratinocytes |
| VIII | Cornea | Corneal fibroblasts |
| IX | Cartilage | |
| X | Hypertrophic cartilage | |
| XI | Cartilage | |
| XII | Papillary dermis | Fibroblasts |
| XIV (undulin) | Reticular dermis | Fibroblasts |
| XVII | P170 bullous pemphigoid antigen | Keratinocytes |

In various embodiments, the cultured three-dimensional tissue has a characteristic repertoire of cellular products produced by the cells, such as growth factors. In some embodiments, the cultured three-dimensional tissues are characterized by the expression and/or secretion of the growth factors shown in Table 2.

| **Table 2** | | |
|---|---|---|
| **Growth Factor** | **Expressed by Q-RT-PCR** | **Secreted Amount Determined by ELISA** |
| VEGF | 8 x 10⁶ copies/ug RNA | 700 pg/10⁶ cells/day |
| PDGF A chain | 6 x 10⁵ copies/ug RNA | |
| PDGF B chain | 0 | 0 |
| IGF-1 | 5 x 10⁵ copies/ugRNA | |
| EGF | 3 x 10³ copies/ug RNA | |
| HBEGF | 2 x 10⁴ copies/ ug RNA | |
| KGF | 7 x 10⁴ copies/ug RNA | |
| TGF-β1 | 6 x 10⁶ copies/ug RNA | 300 pg/10⁶ cells/day |
| TGF-β3 | 1 x 10⁴ copies/ug RNA | |
| HGF | 2 x 10⁴ copies/ug RNA | 1 ng/10⁶ cells/day |
| IL-1a | 1 x 10₄ copies/ug RNA | Below detection |
| IL-1b | 0 | |
| TNF-a | 1 x 10⁷ copies/ ug RNA | |
| TNF-b | 0 | |
| IL-6 | 7 x 10⁶ copies/ug RNA | 500 pg/10⁶ cells/day |
| IL-8 | 1 x 10⁷ copies/ug RNA | 25 ng/10⁶ cells/day |
| IL-12 | 0 | |
| IL-15 | 0 | |
| NGF | 0 | |
| G-CSF | 1 x 10⁴ copies/ug RNA | 300 pg/10⁶ cells/day |
| Angiopoietin | 1 x 10⁴ copies/ug RNA | |

In some embodiments, the cultured three-dimensional tissue is characterized by the expression and/or secretion of connective tissue growth factor (CTGF). CTGF is a well-known fibroblast mitogen and angiogenic factor that plays an important role in bone formation, wound healing, and angiogenesis. See, e.g., Luo, Q., et al., 2004, J. Biol. Chem., 279:55958-68; Leask and Abraham, 2003, Biochem Cell Biol, 81:355-63; Mecurio, S.B., et al., 2004, Development, 131:2137-47; and, Takigawa, M., 2003, Drug News Perspect, 16:11-21*.*

In addition to the above list of growth factors, the three dimensional tissue is also characterized by the expression of Wnt proteins. "Wnt" or "Wnt protein" as used herein refers to a protein with one or more of the following functional activities: (1) binding to Wnt receptors, also referred to a Frizzled proteins; (2) modulating phosphorylation of Dishevelled protein and cellular localization of Axin; (3) modulation of cellular β-catenin levels and corresponding signaling pathway; (4) modulation of TCF/LEF transcription factors; and, (5) increasing intracellular calcium and activation of Ca⁺² sensitive proteins (*e.g*., calmodulin dependent kinase). "Modulation" as used in the context of Wnt proteins refers to an increase or decrease in cellular levels, changes in intracellular distribution, and/or changes in functional (*e.g*., enzymatic) activity of the molecule modulated by Wnt.

Of relevance to the present disclosure are Wnt proteins expressed in mammals, such as rodents, felines, canines, ungulates, and primates. For instance, human Wnt proteins that have been identified share 27% to 83% amino-acid sequence identity. Additional structural characteristics of Wnt proteins are a conserved pattern of about 23 or 24 cysteine residues, a hydrophobic signal sequence, and a conserved asparagine linked oligosaccharide modification sequence. Some Wnt proteins are also lipid modified, such as with a palmitoyl group (Wilkert et al., 2003, Nature 423(6938):448-52). Exemplary Wnt proteins and corresponding genes expressed in mammals include, among others, Wnt 1, Wnt 2, Wnt 2B, Wnt 3, Wnt3A, Wnt4, Wnt 4B, Wnt5A, Wnt 5B, Wnt 6, Wnt 7A, Wnt 7B, Wnt8A, Wnt8B, Wnt9A, Wnt9B, Wnt10A, Wnt11, and Wnt 16. Other identified forms of Wnt, such as Wnt12, Wnt13, Wnt14, and Wnt 15, appear to fall within the proteins described for Wnt 1-11 and 16. Protein and amino acid sequences of each of the mammalian Wnt proteins are available in databases such as SwissPro and Genbank (NCBI). See, also, U.S. Publication No. 2004/0248803 and U.S. application no. 11/216,580, entitled, "Compositions and Methods Comprising Wnt Proteins to Promote Repair of Damaged Tissue," filed August 30, 2005, and U.S. application no. 11/217,121, entitled, "Compositions and Methods for Promoting Hair Growth," filed August 30, 2005.

Various techniques for the isolation and identification of Wnt proteins are known in the art. See, *e.g*., U.S. Publication No. 2004/0248803.

In some embodiments, the Wnt proteins comprise at least Wnt5a, Wnt7a, and Wnt11. As used herein, Wnt5a refers to a Wnt protein with the functional activities described above and sequence similarity to human Wnt protein with the amino acid sequence in NCBI Accession Nos. AAH74783 (gI:50959709) or AAA16842 (gI:348918) (see also, Danielson et al., 1995, J. Biol. Chem. 270(52):31225-34). Wnt7a refers to a Wnt protein with the functional properties of the Wnt proteins described above and sequence similarity to human Wnt protein with the amino acid sequence in NCBI Accession Nos. BAA82509 (gI:5509901); AAC51319.1 (GI:2105100); and 000755 (gI:2501663) (see also, Ikegawa et al., 1996, Cytogenet Cell Genet. 74(1-2):149-52; Bui et al., 1997, Gene 189(1):25-9). Wnt11 refers to a Wnt protein with the functional activities described above and sequence similarity to human Wnt protein with the amino acid sequence in NCBI Accession Nos. BAB72099 (gI:17026012); CAA74159 (gI:3850708); and CAA73223.1 (gI:3850706) (see also, Kirikoshi et al., 2001, Int. J. Mol. Med. 8(6):651-6); Lako et al., 1998, Gene 219(1-2):101-10). As used herein, in the context of specific Wnt proteins, "sequence similarity" refers to an amino acid sequence identity of at least about 80% or more, at least about 90% or more, at least about 95% or more, or at least about 97% or more, or at least about 98% or more when compared to the reference sequence. For instance, human Wnt7a displays about 97% amino acid sequence identity to murine Wnt7a, while the amino acid sequence of human Wnt7a displays about 64% amino acid identity to human Wnt5a (Bui et al., *sepra*).

The expression and/or secretion of various growth factors and/or Wnt proteins by the three-dimensional tissue can be modulated by incorporating cells that release different levels of the factors of interest. For example, vascular smooth muscle cells, are known to produce substantially more VEGF than human dermal fibroblasts. By utilizing vascular smooth muscle cells, instead of, or in addition to fibroblasts, the expression and/or secretion of VEGF by the three dimensional tissue can be modulated.

### 5.4 Genetically Engineered Cells

Genetically engineered three-dimensional tissue can be prepared as described in U.S. Patent No. 5,755,964, the disclosure of which is incorporated herein by reference in its entirety. A genetically-engineered tissue can serve as a gene delivery vehicle for sustained release of growth factors and/or Wnt proteins *in vivo.* For example, in certain embodiments, cells, such as stromal cells, can be engineered to express a gene product that is either exogenous or endogenous to the engineered cell. Stromal cells that can be genetically engineered include, but are not limited to, fibroblasts (of fetal, neonatal, or adult origin), smooth muscle cells, cardiac muscle cells, stem or progenitor cells, and other cells found in loose connective tissue such as endothelial cells, macrophages, monocytes, adipocytes, pericytes, and reticular cells found in bone marrow. In various embodiments, stem or progenitor cells can be engineered to express an exogenous or endogenous gene product, and cultured on a three-dimensional scaffold, alone or in combination with stromal cells.

The cells and tissues can be engineered to express a desired gene product which can impart a wide variety of functions, including, but not limited to, enhanced function of the genetically engineered cells and tissues to promote tissue healing when implanted *in vivo.* The desired gene product can be a peptide or protein, such as an enzyme, hormone, cytokine, a regulatory protein, such as a transcription factor or DNA binding protein, a structural protein, such as a cell surface protein, or the desired gene product may be a nucleic acid such as a ribosome or antisense molecule. In some embodiments, the desired gene product is one or more Wnt proteins, which play a role in differentiation and proliferation of a variety of cells as described above (see, *e.g*., Miller, J.R., 2001, Genome Biology 3:3001.1-3001.15). For example, the recombinantly engineered cells can be made to express specific Wnt factors, including, but not limited to, Wnt5a, Wnt7a, and Wnt11.

In some embodiments, the desired gene products provide enhanced properties to the genetically engineered cells including, but not limited to, gene products which enhance cell growth, *e.g*., vascular endothelial growth factor (VEGF), hepatocyte growth factor (HGF), fibroblast growth factors (FGF), platelet derived growth factor (PDGF), epidermal growth factor (EGF), transforming growth factor (TGF), connective tissue growth factor (CTGF) and Wnt factors. In other embodiments, the cells and tissues are genetically engineered to express desired gene products which result in cell immortalization, *e.g*., oncogenes or telomerese.

In other embodiments, the cells and tissues are genetically engineered to express gene products which provide protective functions *in vitro* such as cyropreservation and anti-desiccation properties, *e.g*., trehalose (U.S. Patent Nos. 4,891,319, 5,290,765, and 5,693,788). The cells and tissues can also be engineered to express gene products which provide a protective function *in vivo,* such as those which would protect the cells from an inflammatory response and protect against rejection by the host's immune system, such as HLA epitopes, MHC alleles, immunoglobulin and receptor epitopes, epitopes of cellular adhesion molecules, cytokines and chemokines.

There are a number of ways that the desired gene products can be engineered to be expressed by the cells and tissues of the present invention. The desired gene products can be engineered to be expressed constitutively or in a tissue-specific or stimuli-specific manner. The nucleotide sequences encoding the desired gene products can be operably linked, *e.g*., to promoter elements which are constitutively active, tissue-specific, or induced upon presence of one or more specific stimulus.

In some embodiments, the nucleotide sequences encoding the engineered gene products are operably linked to regulatory promoter elements that are responsive to shear or radial stress. In these embodiments, the promoter element is activated by passing blood flow (shear), as well as by the radial stress that is induced as a result of the pulsatile flow of blood through the heart or vessel.

Examples of suitable regulatory promoter elements include, but are not limited to, tetracycline responsive elements, nicotine responsive elements, insulin responsive elements, glucose responsive elements, interferon responsive elements, glucocorticoid responsive elements, estrogen/progesterone responsive elements, retinoid acid responsive elements, viral transactivators, early or late promoters of SV40 adenovirus, the lac system, the trp system, the TAC system, the TRC system, the promoter for 3-phosphoglycerate and the promoters of acid phosphatase. In addition, artificial response elements can be constructed, comprising multimers of transcription factor binding sites and hormone-response elements similar to the molecular architecture of naturally-occurring promoters and enhancers (see, *e.g*., Herr and Clarke, 1986, J Cell 45(3): 461-70). Such artificial composite regulatory regions can be designed to respond to any desirable signal and be expressed in particular cell-types depending on the promoter/enhancer binding sites selected.

In some embodiments, the engineered three-dimensional tissue includes genetically engineered cells and produces naturally secreted factors that stimulate proliferation and differentiation of stem cells and/or progenitor cells involved in the revascularization and healing of weakened heart tissue.

### 5.5 Use of Cultured Three-Dimensional Tissues to Treat Congestive Heart Failure

The three-dimensional tissues described herein find use in treating congestive heart failure. The effects of congestive heart failure range from impairment during physical exertion to a complete failure of cardiac pumping function at any level of activity. Clinical manifestations of congestive heart failure include respiratory distress, such as shortness of breath and fatigue, and reduced exercise capacity or tolerance. Clinically, the signs and symptoms manifested by a particular individual are typically classified into one of four groups, Class 1 - Class 4. Class 1 heart failure is the mildest, whereas Class 4 heart failure is the most severe.

Accordingly, the compositions and uses described herein can be administered either alone, or in combination with, conventional treatment regimes to treat individuals classified in one of the four heart failure classes. The ability of the three-dimensional tissue to promote the healing of a heart in an individual clinically manifesting at least one symptom associated with congestive heart failure, depends in part, on the severity of the heart failure, e.g., Class I, II, III, or IV.

Without being bound by theory, application of the three-dimensional tissue to a damaged or diseased heart promotes various biological activities involved in the healing of the heart. By way of example, but not limitation, attachment of the compositions described herein to a damaged or diseased heart can be used to improve the pumping efficiency of the heart.

Example 1 show that cultured three-dimensional tissue sutured onto the surface of the myocardium in the area of ischemia positively influences the events of wound healing in diseased hearts resulting in favorable ventricular remodeling, improved ventricular performance, and improved ventricular wall motion.

The uses and compositions described herein can be used in combination with conventional treatments, such as the administration of various pharmaceutical agents and surgical procedures. For example, in some embodiments, the cultured three-dimensional tissue is administered with one or more of the medications used to treat heart failure. Medications suitable for use in the methods described herein include angiotensin-converting enzyme (ACE) inhibitors (e.g., enalapril (Vasotec), lisinopril (Prinivil, Zestril) and captopril (Capoten)), angiotensin II (A-II) receptor blockers (e.g., losartan (Cozaar) and valsartan (Diovan)), diuretics (e.g., bumetanide (Bumex), furosemide (Lasix, Fumide), and spironolactone (Aldaetone)), digoxin (Lanoxin), beta blockers, and nesiritide (Natrecor) can be used.

Additionally, the cultured three-dimensional tissue can be used with other options used to treat severe heart failure including heart pumps, also referred to as left ventricular assist devices (LVADs), biventricular cardiac pacemakers, cardiac wrap surgery, artificial hearts, and enhanced external counterpulsation (EECP), and cardiac wrap surgery (see, e.g., U.S. Patent Nos. 6,425,856, 6,085,754, 6,572,533, and 6,730,016).

In some embodiments, the cultured three-dimensional tissue is used in conjunction with cardiac wrap surgery. In these embodiments, a flexible pouch or jacket is used to deliver and/or attach the three-dimensional scaffold comprising the three dimensional-tissue. The three-dimensional tissue can be placed inside or embedded within the pouch prior to placement over the damaged or weakened heart tissue. In other embodiments, the pouch and the three-dimensional framework can be joined together. For example, the pouch and the three-dimensional framework can be joined together using a stretchable stitch assembly. In other embodiments, the three-dimensional framework can be configured to comprise threads useful for joining the framework to the pouch. U.S. Patent Nos. 6,416,459, 5,702,343, 6,077,218, 6,126,590, 6,155,972, 6,241,654, 6,425,856, 6, 230,714, 6,241,654, 6,155,972, 6,293,906, 6,425,856, 6,085,754, 6,572,533, and 6,730,016 and U.S. Patent Publication Nos. 2003/0229265, and 2003/0229261,
describe various embodiments of pouches and jackets, *e.g*., cardiac constraint devices, that can be used to deliver and/or attach the three-dimensional stromal tissue.

In some embodiments, other devices, in addition to the three-dimensional tissue are attached to the pouch, *e.g*., electrodes for defibrillation, a tension indicator for indicating when the jacket is adjusted on the heart to a desired degree of tensioning, and used in the methods and compositions described herein. See, *e.g*., U.S. Patent Nos. 6,169,922 and 6,174,279.

As described in more detail below, one or more of the tissues comprising the wall of the heart of an individual diagnosed with one of the disease states described above, can be contacted with a cultured three-dimensional tissue, including the epicardium, the myocardium and the endocardium.

### 5.6 Assays Useful for Determining the Efficacy of Treating CHF with Three-Dimensional Tissue

A number of methods can be used to measure changes in the functioning of the heart in an individual diagnosed with congestive heart failure before and after attachment of the cultured three-dimensional tissue. For example, an echocardiogram can be used to determine the capacity at which the heart is pumping. The percentage of blood pumped out of the left ventricle with each heartbeat is referred to as the ejection fraction. In a healthy heart, the ejection fraction is about 60 percent. In an individual with congestive heart failure caused by the inability of the left ventricle to contract vigorously, *i.e*., systolic heart failure, the ejection fraction is usually less than 40 percent. Depending on the severity and cause of the heart failure, ejection fractions typically range from less than 40 percent to 15 percent or less. An echocardiogram can also be used to distinguish between systolic heart failure and diastolic heart failure, in which the pumping function is normal but the heart is stiff.

Echocardiograms can be used to compare the ejection fractions before and following treatment with the cultured three dimensional tissue. In certain embodiments, treatment with the cultured three-dimensional tissue results in improvements in the ejection fraction between 3 to 5 percent. In other embodiments, treatment with the cultured three-dimensional tissue results in improvements in the ejection fraction between 5 to 10 percent. In still other embodiments, treatment with the cultured three-dimensional tissue results in improvements in the ejection fraction greater than 10 percent.

Nuclear scans, such as radionuclide ventriculography (RNV) or multiple gated acquisition (MUGA) scanning can be used to determine how much blood the heart pumps with each beat. These tests are done using a small amount of dye injected in the veins of an individual A special camera is used to detect the radioactive material as it flows through the heart. Other tests include X-rays and blood tests. Chest X-rays can be used to determine the size of the heart and if fluid has accumulated in the lungs. Blood tests can be used to check for a specific indicator of congestive heart failure, brain natriuretic peptide (BNP). BNP is secreted by the heart in high levels when it is overworked. Thus, changes in the level of BNP in the blood can be used to monitor the efficacy of the treatment regime.

### 5.7 Administration and Dosage of Cultured Three-Dimensional Tissue

A variety of methods can be used to attach and/or contact the cultured three dimensional tissue to the heart of an individual diagnosed with congestive heart failure. Suitable means for attachment include, but are not limited to, direct adherence between the three-dimensional tissue and the heart tissue, biological glue, synthetic glue, lasers, and hydrogel. A number of hemostatic agents and sealants are commercially available, including but not limited to, "SURGICAL" (oxidized cellulose), "ACTIFOAM" (collagen), "FIBRX" (light-activated fibrin sealant), "BOREAL" (fibrin sealant), "FIBROCAPS" (dry powder fibrin sealant), polysaccharide polymers p-G1 cNAc ("SYVEC" patch; Marine Polymer Technologies), Polymer 27CK (Protein Polymer Tech.). Medical devices and apparatus for preparing autologous fibrin sealants from 120 ml of a patient's blood in the operating room in one and one-half hour are also known (*e.g*., Vivostat System).

In some embodiments, the cultured three-dimensional tissue are attached directly to heart tissue via cellular attachment. For example, in some embodiments, the three-dimensional tissue is attached to one or more of the tissues of the heart, including the epicardium, myocardium and endocardium. When attaching a three-dimensional tissue to the heart epicardium or myocardium, typically the pericardium (i.e., the heart sac) is opened or pierced prior to attachment of the three-dimensional tissue. In other embodiments, for example when attaching a three-dimensional tissue to the endocardium, a catheter or similar device is inserted into a ventricle of the heart and the three-dimensional tissue attached to the wall of the ventricle.

In some embodiments, the three-dimensional tissue is attached to heart tissue using a surgical glue. Surgical glues suitable for use in the methods and compositions described herein include biological glues, such as a fibrin glue. For a discussion of applications using fibrin glue compositions see, *e.g*., U.S. Patent Application Serial Number 10/851,938 and the various references disclosed therein.

In some embodiments, a laser is used to attach the three-dimensional tissue to heart tissue. By way of example, a laser dye is applied to the heart, the three-dimensional tissue, or both, and activated using a laser of the appropriate wavelength to adhere the cultured three-dimensional tissue to the heart. For a discussion of various applications using a laser see, *e.g*., U.S. Patent application no. 10/851,938.

In some embodiments, a hydrogel is used to attach the cultured three-dimensional tissue to heart tissue. A number of natural and synthetic polymeric materials can be used to form hydrogel compositions. For example, polysaccharides, *e.g*., alginate, can be crosslinked with divalent cations, polyphosphazenes and polyacrylates ionically or by ultraviolet polymerization (see *e.g*., U.S. Patent No. 5,709,854). Alternatively, a synthetic surgical glue such as 2-octyl cyanoacrylate ("DERMABOND", Ethicon, Inc., Somerville, NJ) can be used to attach the three-dimensional tissue to heart tissue.

In some embodiments, the cultured three-dimensional tissue is attached to heart tissue using one or more sutures as described in U.S. Patent application no. 10/851,938. In other embodiments, the sutures comprise cultured three-dimensional tissue as described in U.S. application no. 11/216,574, entitled "Three Dimensional Tissues and Uses Thereof," filed August 30, 2005.

The cultured three-dimensional tissue is used in an amount effective to promote tissue healing and/or revascularization of weakened or damaged heart tissue in an individual diagnosed with congestive heart failure. The amount of the cultured three-dimensional tissue administered, depends, in part, on the severity of the congestive heart failure, whether the cultured three-dimensional tissue is used as an injectable composition (see, e.g., U.S. application no. 11/216,574, entitled, "Cultured Three Dimensional Tissues and Uses Thereof," filed August 30, 2005), the concentration of the various growth factors and/or Wnt proteins present, the number of viable cells comprising the cultured three-dimensional tissue, and/or ease of access to the heart tissue(s) being treated. Determination of an effective dosage is well within the capabilities of the those skilled in the art. Suitable animal models, such as the canine model described in Example 1, can be used for testing the efficacy of the dosage on a particular tissue of the heart.

As used herein "dose" refers to the number of cohesive pieces of cultured three-dimensional tissue applied to the heart of an individual diagnosed with congestive heart failure. A typical cohesive piece of cultured three-dimensional tissue is approximately 35 cm². As will be appreciated by those skilled in the art, the absolute dimensions of the cohesive piece can vary, as long it comprises a sufficient number of cells to stimulate angiogenesis and/or promote healing of weakened or damaged heart tissue in an individual diagnosed with congestive heart failure. Thus, cohesive pieces suitable for use in the methods described herein can range in size from 15 cm² to 50 cm².

The application of more than one cohesive piece of cultured three-dimensional tissue can be used to increase the area of the heart treatable by the methods described herein. For example, in embodiments using a two pieces of cohesive tissue, the treatable area is approximately doubled in size. In embodiments using three cohesive pieces of cultured three-dimensional tissue, the treatable area is approximately tripled in size. In embodiments using four cohesive pieces of cultured three-dimensional tissue, the treatable area is approximately quadrupled in size. In embodiments using five cohesive pieces of cultured three-dimensional tissue, the treatable area is approximately five-fold, i.e. from 35 cm² to 175 cm².

In some embodiments, one cohesive piece of cultured three-dimensional tissue is attached to a region of the heart in an individual diagnosed with congestive heart failure.

In other embodiments, two cohesive pieces of cultured three-dimensional tissue are attached to a region of the heart in an individual diagnosed with congestive heart failure.

In other embodiments, three cohesive pieces of cultured three-dimensional tissue are attached to a region of the heart in an individual diagnosed with congestive heart failure.

In other embodiments, four, five, or more cohesive pieces of cultured three-dimensional tissue are attached to a region of the heart in an individual diagnosed with congestive heart failure.

In embodiments in which two or more cohesive pieces of cultured three-dimensional tissue are administered, the proximity of one piece to another can be adjusted, depending in part on, the severity of the congestive heart failure, the extent of the area being treated, and/or ease of access to the heart tissue(s) being treated. For example, in some embodiments, the cultured pieces of three-dimensional tissue can be located immediately adjacent to each other, such that one or more edges of one piece contact one or more edges of a second piece. In other embodiments, the pieces can be attached to the heart tissue such that the edges of one piece do not touch the edges of another piece. In these embodiments, the pieces can be separated from each other by an appropriate distance based on the anatomical and/or disease conditions presented by the patient. Determination of the proximity of one piece to another, is well within the capabilities of the those skilled in the art, and if desired can be tested using suitable animal models, such as the canine model described in Example 1.

In embodiments that comprise a plurality of pieces of cultured three-dimensional tissue, some, or all of the pieces can be attached to the same or different areas of the heart.

In embodiments that comprise a plurality of pieces of cultured three-dimensional tissue, the pieces are simultaneously attached, or concurrently attached to the heart.

In some embodiments, the pieces are administered over time. The frequency and interval of administration depends, in part, on the severity of the congestive heart failure, whether the cultured three-dimensional tissue is used as an injectable composition (see, e.g., U.S. application no. 11/216,574, entitled, "Cultured Three Dimensional Tissues and Uses Thereof," filed August 30, 2005), the concentration of the various growth factors and/or Wnt proteins present, the number of viable cells comprising the cultured three-dimensional tissue, and/or ease of access to the heart tissue(s) being treated. Determination of the frequency of administration and the duration between successive applications is well within the capabilities of the those skilled in the art, and if desired, can be tested using suitable animal models, such as the canine model described in Example 1.

In some embodiments, the cultured three-dimensional tissue is administered as an injectable composition as described in the U.S. application no. 11/216,574, entitled, "Cultured Three Dimensional Tissues and Uses Thereof," filed August 30, 2005). Guidance for the administration and effective dosage of injectable compositions for the treatment of ischemic tissue is provided in U.S. application no. 11/216,574, entitled, "Cultured Three Dimensional Tissues and Uses Thereof," August 30, 2005).

### 6. EXAMPLES

### Example 1: Treatment of Chronically Ischemic Tissue in a Dog Heart Study

The three-dimensional cultured tissue, i.e., Anginera™ (also referred to herein as Dermagraft™), was manufactured by Smith & Nephew. Anginera™ is a sterile, cryopreserved, human fibroblast-based tissue generated by the culture of human neonatal dermal fibroblasts onto a bioabsorbable polyglactin mesh scaffold (Vicryl™). The process is carried out within a specialized growth container or bioreactor. Tissue growth is supported with cell medium that provides the required nutrients for cell proliferation. The closed bioreactor system used to manufacture Anginera™ maintains a controlled environment for the growth of a sterile, uniform and reproducible, viable human tissue.

The dermal fibroblasts used in the manufacture of Anginera™ were obtained from human neonatal foreskin tissue derived from routine circumcision procedures. Every lot of Anginera™ passes USP sterility tests before being released for use. It is cryopreserved at -75°C after harvest to provide an extended shelf life. Following thawing, about 60% of the cells retain viability and are capable of secreting growth factors, matrix proteins, and glycosaminoglycans.

A canine study was used to evaluate the safety and efficacy of Anginera™ for treating chronically ischemic heart tissue. Evaluation of the data from the canine study demonstrated Anginera™ to be safe at all dosing levels. The canine study was in compliance with the Food and Drug Administration Good Laboratory Practice Regulations (GLP) as set forth in Title 21 of the U.S. Code of Federal Regulations, Part 58.

Initially, chronic myocardial ischemia was induced in forty animals (four groups of five male and five female mongrel dogs) through the surgical placement of an ameroid constrictor on the ventral interventricular branch of the left anterior descending coronary artery (LAD). Approximately 30 days (± two days) following the surgical placement of an ameroid constrictor, the animals received one of four treatments: Group 1, sham surgical treatment; Group 2, surgical application of one unit of non-viable Anginera™; Group 3, surgical application of one unit of viable Anginera™; and, Group 4, surgical application of three units of viable Anginera™. Anginera™ used in this study was Dermagraft™ released by Smith & Nephew for clinical use. All investigators performing tests or analyzing data were blinded to the greatest extent possible as to the identity of an animal's treatment. Two animals per sex were necropsied on Day 30 (± one day), and three animals per sex from each treatment group were necropsied on Day 90 (± one day) (see Table III).

Electrocardiograms and direct arterial pressure were continuously monitored during the surgical procedure. A left lateral thoracotomy was performed between the fourth and fifth ribs. Prior to heart isolation, lidocaine was given intravenously (2 mg/kg) and topically as needed to help control arrhythmias. The heart was isolated and a pericardial well was constructed. The ventral interventricular branch of the left anterior descending coronary artery (LAD) was identified and isolated for placement of an ameroid constrictor. The appropriately sized ameroid constrictor (Cardovascular Equipment Corporation, Wakefiled, Massachusetts, 2.0-3.0 mm) was placed around the proximal portion of the LAD. Any ventricular arrhythmias were treated using pharmacological agents, i.e., lidocaine, dexamethasone, bretyllium, as needed and as indicated. The study design is illustrated in Table 3.

| **Table 3** | | | | | | |
|---|---|---|---|---|---|---|
| **Group Number** | **Number of Animals** | | **Treatment** | | **Treatment Regimen** | **Necropsy Day** |
| | **Males** | **Females** | | | | |
| 1 | 5 | 5 | Ischemia | none | none | Day 30: 2/sex/group |
| | | | only | | | Day 90: 3/sex/group |
| 2 | 5 | 5 | One piece | ~ 35 cm² | NA | Day 30: 2/sex/group |
| | | | non-viable Anginera™ | | | Day 90: 3/sex/group |
| 3 | 5 | 5 | One piece | ~ 35 cm² | Day 1 only | Day 30: 2/sex/group |
| | | | Anginera™ | | | Day 90: 3/sex/group |
| 4 | 5 | 5 | Three pieces | ~ 105cm² | Day 1 only | Day 30: 2/sex/group |
| | | | Anginera™ | | | Day 90: 3/sex/group |

Safety was assessed by evaluating clinical observations, physical and ophthalmic examinations, body weights, body temperatures, cardiac monitoring (including electrocardiography (ECG), arterial blood pressure, heart rate, and echocardiographic determination of left ventricular function), clinical pathology (including hematology, coagulation, serum chemistry, Troponin T, and urinalysis), anatomic pathology and histopathology of selected organs and tissues. Additional evaluation of the echocardiography data from all treatment groups at the Day 30 and Day 90 time points was performed. Finally, a separate analysis of heart histology was performed.

Echocardiograms were collected within four weeks prior to Day -30 (i.e., 30 days prior to surgery, surgery was done at Day 0), approximately eight days prior to Day 1, and approximately eight days prior to sacrifice/necropsy (Day 30 or 90). Trans-thoracic resting and stress echocardiography were performed using methods to standardize echocardiographic windows and views. Animals were manually restrained as much as possible and placed in right lateral recumbency (right side down). Echocardiographic evaluation was performed after the animals have achieved a stable heart rate followed by a second echocardiographic examination under dobutamine-induced increased heart rate. Dobutamine was administered intravenously starting at five micrograms/kg/min and titrated to a maximum infusion rate of 50 micrograms/kg/min to achieve 50% increase in heart rate (± 10%). Animal ID numbers, study dates, and views were annotated on the video recording of each study. All echo studies were recorded on videotape and images and loops were captured digitally and saved to optical disc. Short-axis images were recorded on both videotape and digitally on optical disc and included at least two cardiac cycles. Segmental contractility, measured as wall thickening (in centimeters), was quantified in the ischemic region and the control region of the left ventricle. These measurements were performed in three cross sectional planes to include basal plane, mid papillary plane and a low-papillary plane. Left ventricular dimensional measurements were taken from 2 dimensional images. Two-chambered and four-chambered long axis images were recorded for the determination of left ventricular volumes, ejection fraction, and cardiac output. The mathematical model for this determination was the biplane, modified Simpsons approximation. Electrocardiograms were recorded coordinate with the echocardiography.

Images saved to optical disc were stored on CDs in a DICOM image format for review in chronological order of the study by at least one board-certified veterinarian cardiologist (VetMed), blinded as to the identity of the samples.

Three measurements were performed on all echocardiographic data and reported as a mean of the three measurements.

Echocardiography was performed on all animals within four weeks prior to ameroid placement. Any animal identified with congenital heart disease or abnormal left ventricular function by echocardiogram was excluded from the study. Dobutamine stress echocardiography was performed to establish baseline comparisons.

Echocardiography was performed on all animals following surgery and placement of the ameroid constrictor on the LAD, and within approximately eight days prior to treatment application. Regional left ventricular wall motion was assessed under resting and dobutamine stress conditions. Any animals identified with transmural infarcts were excluded from the study.

Echocardiography was performed on all animals within approximately eight days of necropsy. Regional left ventricular wall motion was assessed under resting and dobutamine stress conditions. Global left ventricular function was assessed using a combination of left ventricular dimensional measurements, left ventricular volume determinations, ejection fraction, and cardiac output determinations.

A separate non-GLP echocardiography analysis was performed on the original echocardiography data to provide statistical comparisons of selected parameters. One-way analysis of variance (ANOVA) was used to determine a significant difference (p<0.05) between treatment groups. Comparisons were made between and within groups with specific focus on parameter changes under resting conditions vs. dobutamine-stress conditions at both the 30 and 90 day time points. The parameters that were identified for comparison were:
1. Cardiac Output (CO): CO was expected to increase from resting to stress conditions. It is expected that diseased hearts would demonstrate a compromised ability to increase CO under dobutamine-stress conditions.
2. Left Ventricular Ejection Fraction (LVEF): LVEF was also expected to increase from resting to stress conditions. It is expected that diseased hearts would demonstrate a compromised ability to increase LVEF under dobutamine-stress conditions.
3. Left Ventricular End Diastolic Volume Index (LVEDVI): LVEDVI was expected to increase from resting to stress conditions. It is expected that diseased hearts would demonstrate greater increases in LVEDVI under dobutamine-stress conditions.
4. Left Ventricular End Systolic Volume Index (LVESVI): LVESVI was expected to decrease from resting to stress conditions. It is expected that diseased hearts would demonstrate a compromised ability to decrease LVESVI under dobutamine-stress conditions.
5. Systolic Wall Thickening (SWT): SWT values were expected to increase from resting to stress conditions. It is expected that diseased hearts would demonstrate a compromised ability to increase SWT values under dobutamine-stress conditions.

None of the animals observed during the baseline pre-ameroid echo evaluation demonstrated significant left ventricular dysfunction or congenital heart disease. At baseline resting conditions all animals were evaluated to be within normal species ranges for hemodynamic values and wall dimensions. These data demonstrate that animals from all groups began the study with normal range values of left ventricular function. Furthermore, pre-treatment, post-ameroid left ventricular wall dimensions demonstrated a blunted response to dobutamine stress at the basilar (mitral valvular), high papillary, and low papillary levels in comparison to the pre-ameroid baseline assessment, demonstrating diminished wall function in the anterior and lateral wall of the left ventricle. These pre-treatment, post-ameroid echo observations are consistent with the ameroid experimental model that resulted in mild left ventricular dilation secondary to ventricular ischemia and demonstrate that the ameroid canine model used in this study was successful at creating ventricular ischemia measurable by echocardiography.

No animal deaths were observed during the in-life phase of the study.

Clinical observations common to the surgical procedures associated with the exposure of the heart via thoracotomy (ameroid placement) or sternotomy (test article placement) were observed (e.g., swelling, erythema, open incisions, abrasions, etc.). The distribution and frequency of these clinical observations prior to Day 1 was similar between the final treatment groupings. Following the Day 1 surgical administration of treatment, clinical observations were similar between the four treatment groups with the exception that more animals in Groups 1, 2, and 4 (ischemia only, nonviable Anginera^{TM} and three piece Anginera™ respectively), in comparison to Group 3 (single Anginera^{TM}) were observed with open surgical incisions. Most of the open incisions were observed following the sternotomy procedure (application of treatment), although one to two animals per group had open incisions observed following the thoracotomy procedure (application of ameroid occluder). All animals with open incisions were treated with antibiotics until the incisions were closed or had granulated and were dry.

Ophthalmologic examinations, physical examinations, body weights, body temperatures, hematology, coagulation, serum chemistry, Troponin T, urinalysis, surgical hemodynamic/cardiovascular monitoring, and weekly cardiovascular monitoring were all evaluated to be within normal species ranges and were not different between the four groups of animals. Collectively, these data demonstrate the safety of Anginera™ at all dosing levels within the parameters evaluated. Qualitative evaluation of the ECGs demonstrated normal cardiac rhythms for all but three animals (two Group 2 animals and one Group 3 animal). The arrhythmias or conduction disturbances observed in these three animals were evaluated to be either normal variants in dogs or a temporary residual effect of the surgical placement of the test article onto the myocardial surface.

Gross macroscopic pathology observations were limited to numerous myocardial adhesions (between the heart and the pericardium and the pericardium and the lungs or chest wall) and nodular lesions or discolorations in the myocardial tissue surrounding the ameroids. No differences were detected in the frequency or intensity of these observations amongst the four treatment groups of animals. These types of gross observations are consistent with the surgical procedures utilized in this experimental protocol (i.e., thoracotomy and sternotomy). Microscopic pathology observations associated with the surgical placement of the test material included widespread fibrous thickening of the epicardium, correlating to the adhesions between the epicardial surface of the heart and the pericardium or lung, and limited serosanguineous exudates. These observations were noted in all animals in each of the four treatment groups and were felt to be related to the surgical procedures and not to specific treatment with the test article. Therefore, no safety concerns were evident from the histologic results, and the tissue responses observed were consistent with tissue injury attributed to the surgical procedures. Microscopic changes noted to be a result of the surgical placement of the ameroid occluder onto the coronary artery ranged from low-grade lymphoplasmacytic and histiocytic infiltrates, varying degrees of arterial intimal hyperplasia in the ameroided vessel, and areas of myocardial infarction. Transmural infarction was not observed in any of the tissue samples examined. Overall, no trends in the incidence or severity of infarction could be associated with specific treatment at the Day 30 or Day 90 evaluation time points.

In summary, evaluation of the primary safety endpoints (including hemodynamic, electrocardiographic, echocardiographic, and clinical and gross pathology observations) demonstrated the safety of Anginera™ at all dosing levels and at both time points.

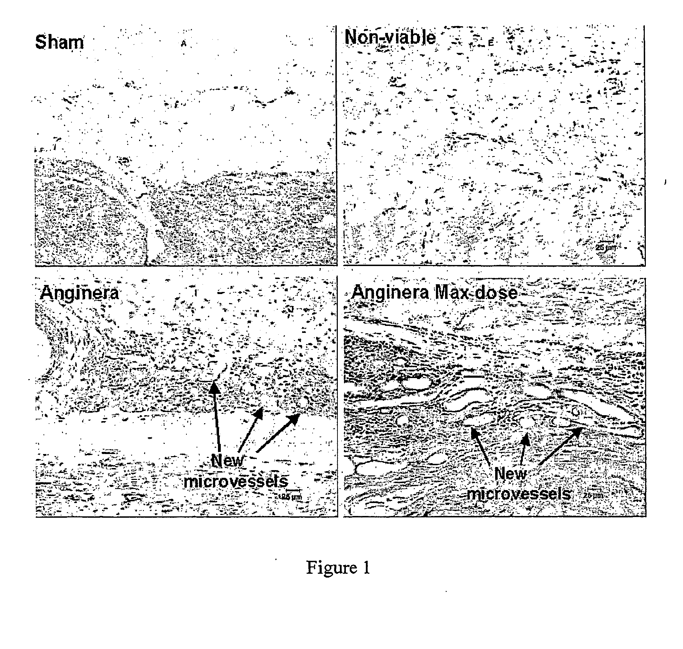

Additional evaluations of heart histology were performed to identify evidence of new microvessel formation. These findings confirm previously reported and published findings of new microvessel formation with the presence of a mature microvasculature (arterioles, venules, and capillaries) (see FIG. 1).

Hematoxylin and eosin stained sections from the canine study were further analyzed to evaluate the cellular infiltrate in association with Anginera™ and the epicardial tissue. This analysis was performed on tissues that were in direct contact with the Anginera™ material. The following observations were made:
1. Scarring indicative of subendocardial ischemic damage was seen in all groups.
2. Group 1 (ischemia only) specimens showed minimal focal pericardial thickening without inflammation.
3. Group 2 (non-viable Anginera™) implants had diffuse mild and focally increased pericardial thickening with minimal inflammation and focal mesothelial proliferation.
4. Groups 3 (single dose Anginera™) and Group 4 (three pieces of Anginera™) had fibrous pericardial thickening with varying amounts of moderate, focal, multifocal or band-like inflammation between the patch and the epicardium, and focal foreign body reaction (most associated with sutures).
5. Less inflammation was seen at 90 than 30 days.
6. No definitive evidence of immunological reaction was seen.
7. In no case was there inflammation involving the myocardium.
8. Increased vasculature was seen focally in areas of pericardial inflammation.

These histopathological evaluations demonstrated no evidence of an immunologic reaction to Anginera™. There was a transient inflammatory response observed in all four treatment groups associated with the experimental conditions. In the viable Anginera™ groups there was evidence of a cellular response, which included an increase in microvasculature specific to the epicardium and pericardium. There was no evidence of a localized fibrosis, associated with the treatment, in the epicardium or myocardium that might lead to arrhythmias. The infiltrates had the morphologic appearance of macrophagic rather than lymphocytic cell types.

Prenecropsy echocardiographic assessment performed as part of the GLP study demonstrated dose-dependant decreases in left ventricular chamber volumes. Resting stroke volume and cardiac output indices were decreased in Group 3, but these mild decreases normalized in response to dobutamine infusion. Resting stroke volume and cardiac output indices decreased in Group 4 while decreases in left ventricular chamber volumes were marked compared with pretreatment values and was diminished over baseline values. These changes were more dramatic in Group 4 compared with Group 3. The response to dobutamine infusion in terms of percent difference in Group 4 was actually better than that seen in baseline values. Stroke volume and cardiac output indices did not return to normal baseline values, but were very close.

Group 3 animals (one unit dose Anginera™) at the 30-day prenecropsy time point had larger left ventricles than Group 3 animals at the 90-day prenecropsy time point or Group 4 animals (three units dose Anginera™) at either the 30 or 90-day prenecropsy time point. Group 4 animals had smaller left ventricles than Group 1, 2, or 3 animals. Compensatory mechanisms in and of themselves cause a decrease in left ventricular size (volume) as was seen in Group 1 untreated animals and Group 2 non-active Anginera™ treated animals. However, the fact that the left ventricular volumes were actually smaller in Group 4 animals than Group 1, 2, or 3 animals suggests a positive treatment effect. Decreases in left ventricular sizes/volumes are at least in part responsible for the decreases in stroke volume index (SVI) and cardiac output index (COI). These decreases returned both cardiac output index and stroke volume index to values similar to or better than normal baseline values that were also improved compared to the pre-treatment values. The most improved function compared with pre-treatment values was in Group 4 animals at the 90-day prenecropsy time point.

As part of the GLP study, segmental wall dimensions and segmental functional data suggested that application of treatment Groups 2, 3, and 4, increased wall dimensions where applied. It also suggested that in these regions there was a mild myocardial stiffening effect-evident in Group 2 dogs that received non-active test article alone. Data from this group also suggests that the non-active test article alone may cause an improvement in overall segmental function in adjacent segments. This may simply be a manifestation of compensatory responses in other segments. Group 3 animals demonstrated either mild increases in segmental function or no change over pretreatment values supporting the fact that Anginera™ was safe and at this dose mildly improved function in ischemic segments, but did not return segmental function to baseline normal values. Group 4 measurements at the basilar level demonstrated increased segmental function with return to close to baseline values and marked improvement over pretreatment values. This was not the case at the high papillary muscle level nor apical levels where segmental wall thickening was mildly depressed in response to dobutamine infusion in most segments. Segments that revealed mildly depressed segmental function had systolic wall dimensions that were increased significantly over either pretreatment values or normalized in response to treatment

A separate non-GLP evaluation of left ventricular EDVI values was performed for two reasons. First, to specifically understand the changes in EDVI values following treatment; and, second, to evaluate the 30 day ameroid period with respect to the canine model. Studies reported in the published literature have suggested that the canine is capable of significant collaterization of the coronary circulation. This can present limitations on the interpretation of functional data purposed to evaluate the benefit of a treatment. However, the canine model remains a well-established model within the published literature.

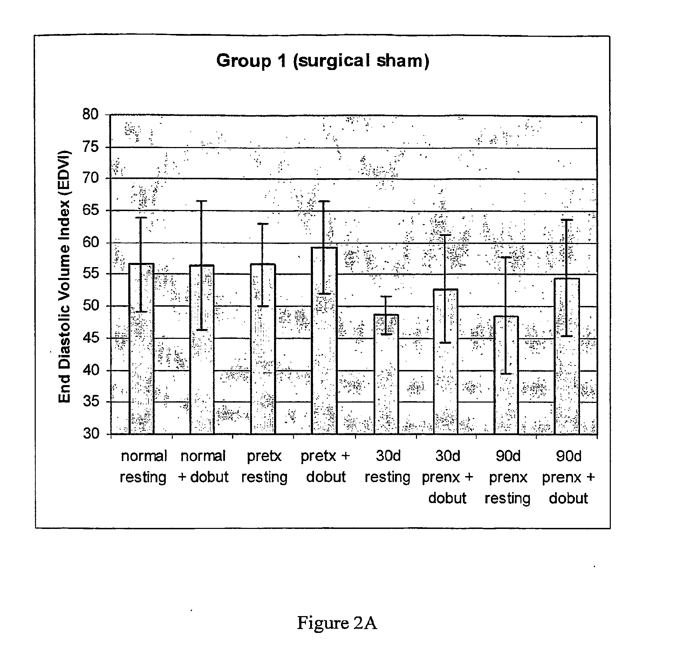

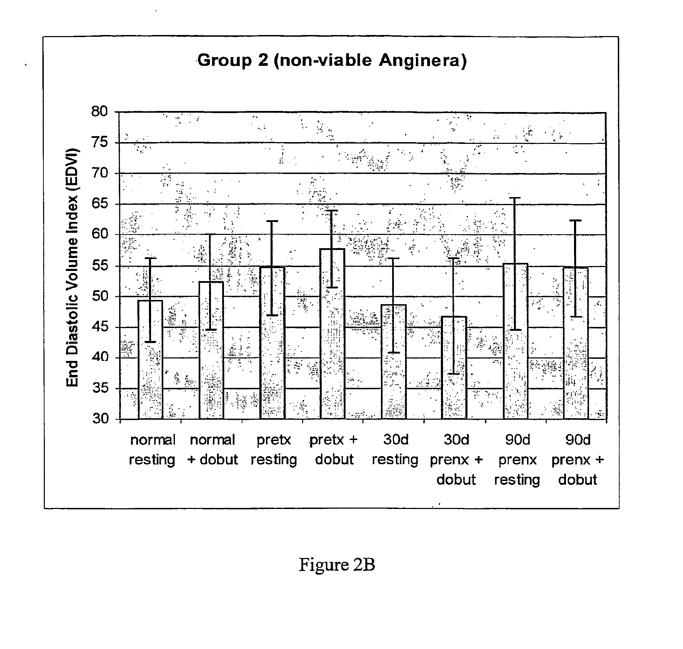

In light of these understandings of the canine model, EDVI parameters were evaluated in more detail. The parameters of EDVI during the 30 day ameroid period appear to suggest that animals treated with a single piece of Anginera™ (Group 3) and those treated with three pieces of Anginera™ (Group 4) may have had a more severe disease condition as suggested by EDVI values at the pre-treatment time point under dobutamine stress (FIG. 2). However, no statistically significant differences were seen in comparisons between these baseline EDVI values, possibly due to the large standard deviations and low sample size. Key to the X axis legend: Normal = pre-ameroid occlusion time point (-30 days); PreTx = ameroid occlusion, pre treatment time point (0 days); 30d PreNx = treatment after 30 days, prior to necropsy (30 days), and 90d PreNx = treatment after 90 days, prior to necropsy (90 days).

As previously described, a separate, secondary evaluation was performed on the original raw data that was collected in the primary echocardiography evaluation. The secondary evaluation focused on specific statistical comparisons of clinically relevant echocardiographic parameters. The general findings of the primary echo evaluation and the specific findings of the secondary echocardiography evaluation support of each other. In addition, in the secondary echocardiography evaluation, parameters of cardiac output (CO), left ventricular ejection fraction (LVEF), left ventricular end systolic volume index (LVESVI), and systolic wall thickening (SWT) support the conclusion that Anginera^{TM} stimulates a positive biologic effect on chronically ischemic canine hearts. Furthermore, these data support the conclusion that treatment with viable Anginera™ improves ventricular performance and ventricular wall motion in chronically ischemic canine hearts after 30 days of treatment.

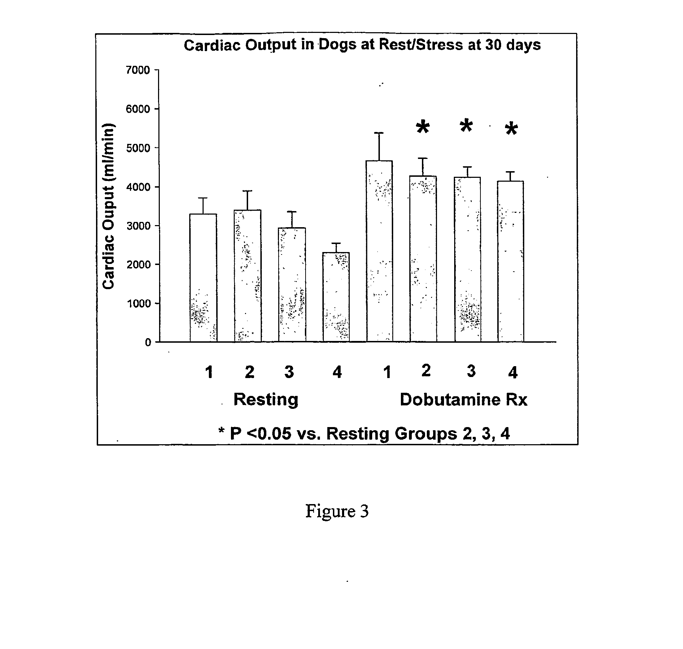

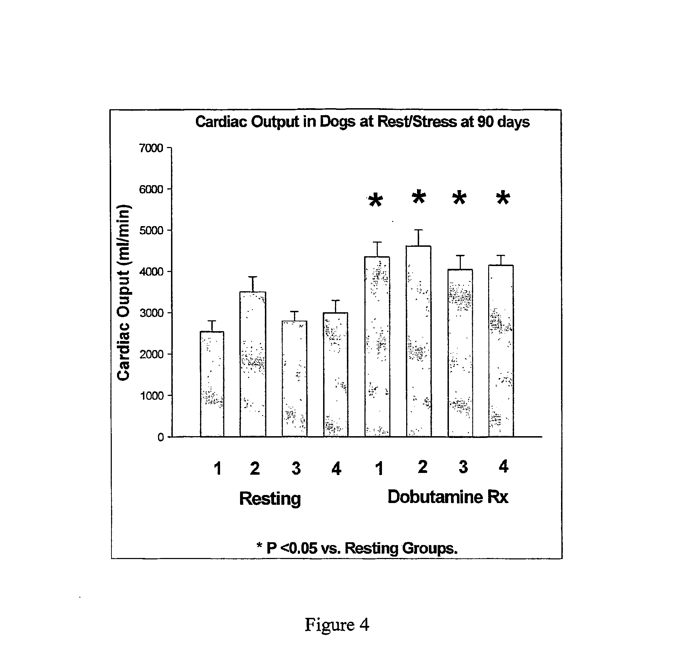

Following 30 days of treatment, dogs in the non-viable, single and multiple Anginera™ patch groups showed a significant (P<0.05) improvement in cardiac output with dobutamine (4273 ± 450,4238 ± 268, and 4144 ± 236 ml/min, respectively) compared to their baseline, resting cardiac output. The sham surgical group did not significantly improve its CO with dobutamine infusion. However, at 90 days all dogs improved their CO with dobutamine, including the sham operated animals (FIGS. 3 and 4). CO was expected to increase from resting to stress conditions. It is expected that diseased hearts would demonstrate a compromised ability to increase CO under dobutamine-stress conditions. These data suggest that dogs treated with non-viable, single, and multiple pieces of Anginera™ had a better CO response to dobutamine than the control sham group at 30 days. By 90 days, all groups performed statistically equivalent to each other.

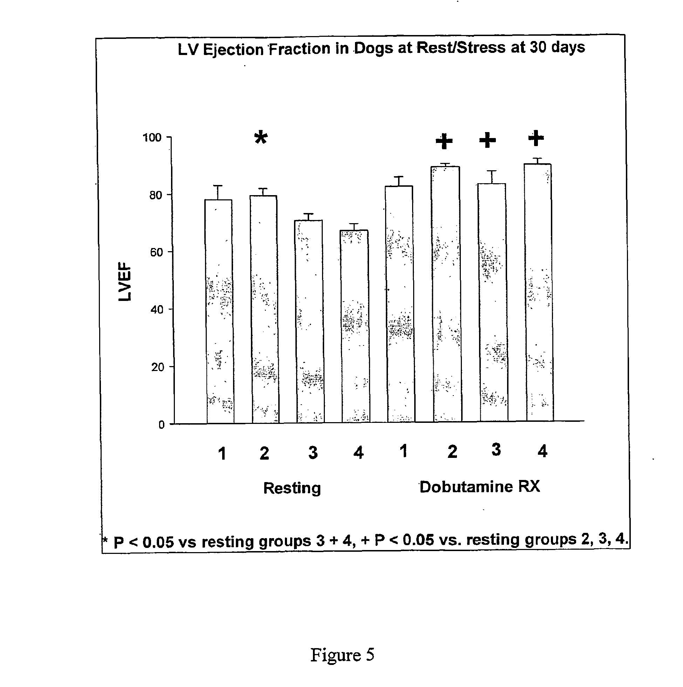

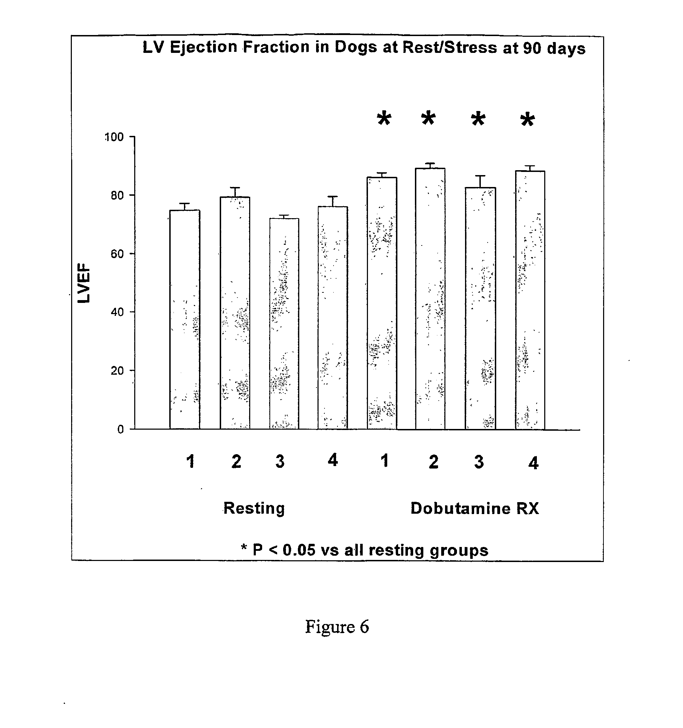

LVEF demonstrated a similar stress response to dobutamine as CO at 30 and 90 days (FIGS. 5 and 6). Specifically after days of treatment, dogs in the non-viable, single and multiple Anginera™ patch groups showed a significant (P<0.05) improvement in LVEF with dobutamine compared to their baseline, resting LVEF. The sham surgical group did not significantly improve its LVEF with dobutamine infusion. However, at 90 days all dogs improved their LVEF with dobutamine, including the sham operated animals. LVEF was expected to increase from resting to stress conditions. It is expected that diseased hearts would demonstrate a compromised ability to increase LVEF under dobutamine-stress conditions. These data suggest that dogs treated with non-viable, single, and multiple pieces of Anginera™ had a better LVEF response to dobutamine than the control sham group at 30 days. By 90 days, all groups performed statistically equivalent to each other.

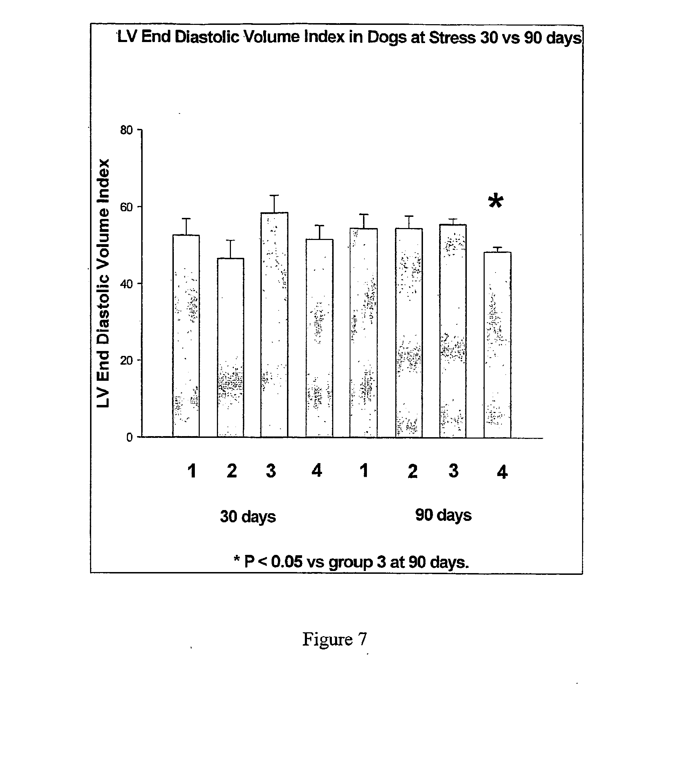

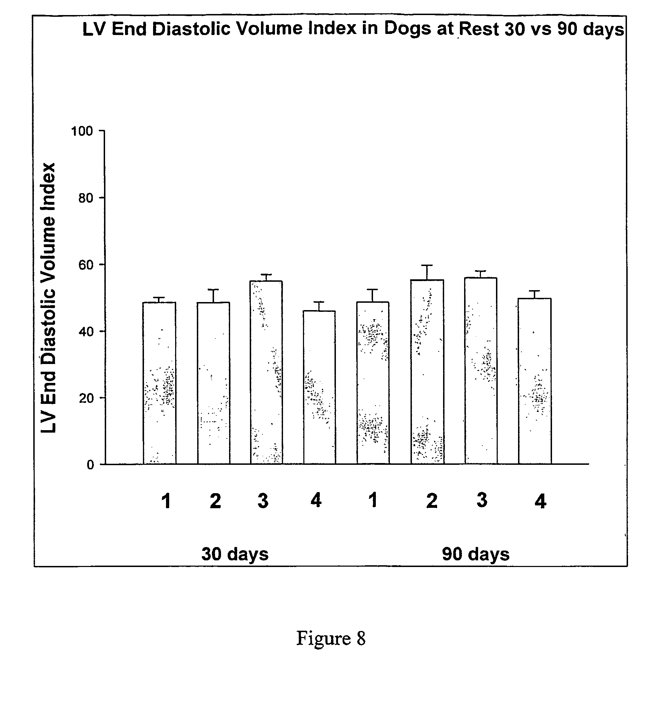

The LVEDV index was measured at rest and during stress in all groups at 30 and 90 days. At rest the LVEDV index was similar in all groups at 30 and 90 days. However, during stress at 90 days there is a significant (P<0.05) decrease in LVEDV index at the highest Anginera™ dose (Group 4) (FIGS. 7 and 8). LVEDVI was expected to increase from resting to stress conditions. It is expected that diseased hearts would demonstrate greater increases in LVEDVI under dobutamine-stress conditions. Therefore, the result of Group 4 animals at 90 days under dobutamine stress having significantly lower LVEDV index values suggests that the maximum treatment group (three pieces of Anginera™) provides additional benefit to the ischemic heart.

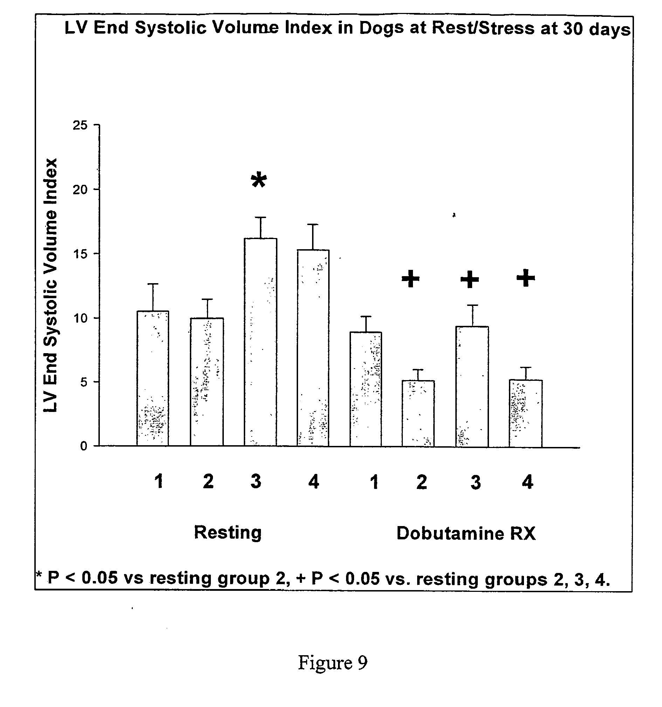

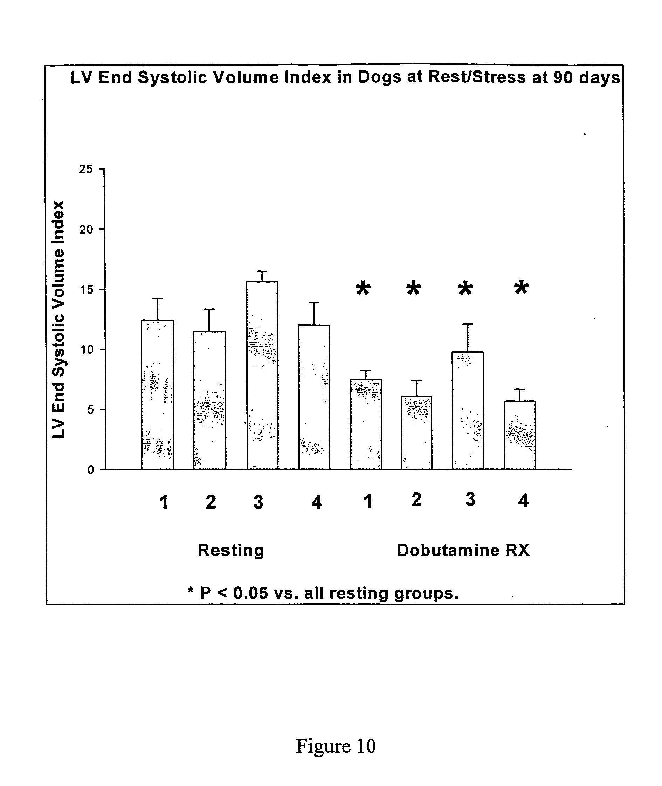

Consistent with the data from LVEF and CO, LVSV index values also significantly decreased with either viable or non-viable Anginera™ at stress compared to baseline at 30 days. At 90 days, there was also an improvement in the LVSV index with the sham surgery animals (FIGS. 9 and 10). LVESV index was expected to decrease from resting to stress conditions. It is expected that diseased hearts would demonstrate a compromised ability to decrease LVESVI under dobutamine-stress conditions. These data suggest that dogs treated with non-viable, single, and multiple pieces of Anginera^{TM} had a better LVESV index response to dobutamine than the control sham group at 30 days. By 90 days, all groups performed statistically equivalent to each other.

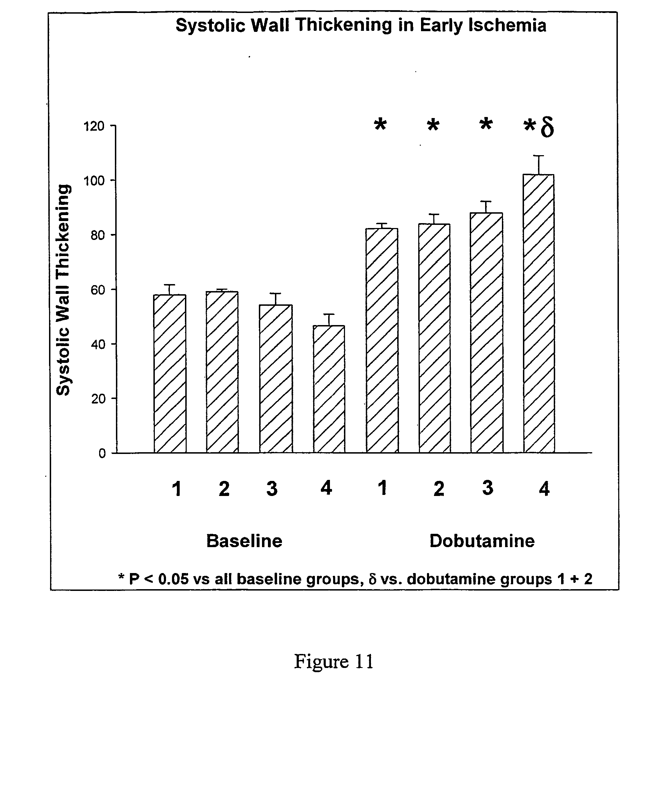

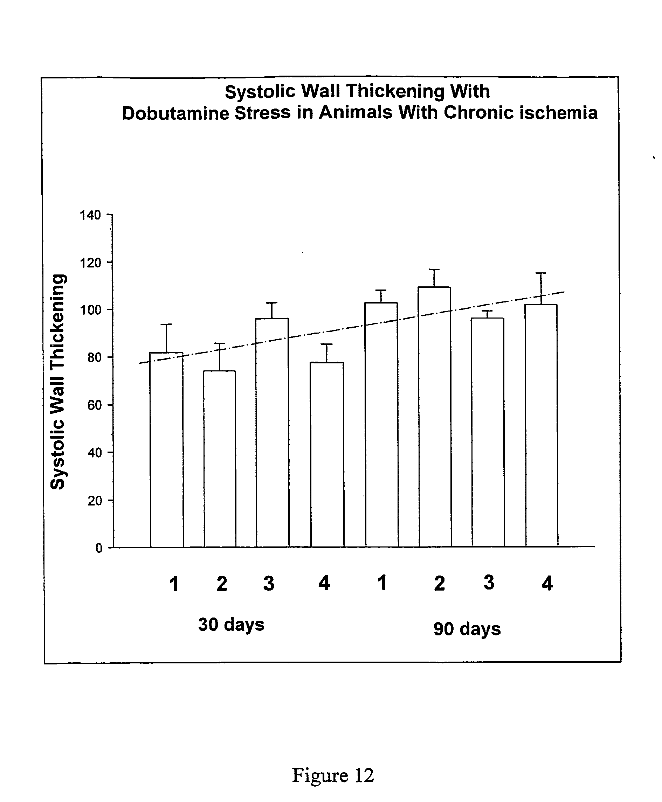

During the early ischemia period, dobutamine increased (P<0.05) SWT in all 4 randomized groups, however there appears to be a dose-dependent relationship since the most significant increase in SWT occurred in dogs that had the three patches of Anginera™ implanted (FIG. 11). At 30 and 90 days post treatment, within the chronic ischemia period, there is a gradual trend demonstrating increasing SWT in response to dobutamine over time, as plotted through a linear regression analysis of all data points (FIG. 12). This appears to occur in both untreated ischemic animals as well as those treated with Anginera™ patches. SWT values were expected to increase from resting to stress conditions. It is expected that diseased hearts would demonstrate a compromised ability to increase SWT values under dobutamine-stress conditions. These data suggest that during the early ischemia period (30 days after treatment), dobutamine increases (P<0.05) SWT in all 4 treatment groups; however, there appears to be a dose-dependent relationship since the most significant increase in SWT occurred in dogs that had the three patches of Anginera™ implanted.

The placement of either non-viable or viable Anginera™ patches, irrespective of the number of patches implanted resulted in an improved LV ejection fraction, increased cardiac output and reduced LV systolic volume index during stress with dobutamine at 30 days after induction of ischemia. In the chronic ischemia animals (group 1), this response was only seen at 90 days; at this time point the chronic ischemia animals were able to mount a response to dobutamine even though they had not received the Anginera™ treatment. This finding is congruent to the published literature where the canine model is described as a model that has an intrinsic ability for coronary collateralization.

In conclusion, the general findings of the primary echo evaluation as part of the GLP study and the specific findings of the separate non-GLP echocardiography analyses are in support of one another. In addition in the separate non-GLP echocardiography analyses, changes in CO, LVEF, LVESVI, and SWT support the conclusions that treatment with Anginera™ improves ventricular performance and ventricular wall motion in chronically ischemic canine hearts after 30 days of treatment.

### Example 2: Injection of Three-Dimensional Stromal Tissues in an Ischemic Mouse Hind Limb Model

The mouse hindlimb ischemia model was developed in two different strains, the C57BL/6 and Balb/C. This model consists of ligating the artery and vein proximal to the bifurcation of the arteria profunda femoris and again at a site 5-7 mm distal. While both strains have been used in the literature, the Balb/C mouse has been found to collateralize less than other strains. Therefore, the Balb/C mouse strain was chosen for further studies.

The mouse hindlimb ischemia model was used to evaluate the ability of minimally invasive constructs to induce angiogenesis *in vivo* when implanted into ischemic peripheral tissues. Ischemia was induced in two animals and the animals injected with a composition of smooth muscle cells cultured on Alkermes® beads. For a control, ischemia was induced in the animals but left untreated. Cells cultured on beads were successfully injected through a 24 gauge Hamilton syringe; however, approximately 50% of the bead volume remained in the syringe (FIGS. *A and *B). All 20 µl of media was injected with approximately 10 µl of the 20 µl of bead volume being delivered into the ischemic muscle. To insure full delivery, pharmaceutically suitable delivery agents such as PEG hydrogels may be used to increase viscosity of the vehicle and provide greater bead delivery.

Observations 2 week after implantation demonstrated evidence of limited new microvessel formation (black arrows) in ischemic limbs treated with smooth muscle cells on Alkermes beads (FIGS. *A and *B) in comparison to control animals with ischemia-only limbs (FIGS. *A and *B).

Studies using the mouse hindlimb ischemia model were also carried out with three dimensional tissues prepared using braided threads. Results suggest the presence of new microvessel formation surrounding the implants after 14 days of implantation (FIGS. *A and *B).

The foregoing descriptions of specific embodiments of the present disclosure have been presented for purposes of illustration and description. They are not intended to be exhaustive or to limit the scope of the disclosure to the precise forms disclosed, and many modifications and variations are possible in light of the above teaching.

## Claims

1. A cultured three-dimensional tissue comprising living fibroblasts for use in the treatment of congestive heart failure in a patient.

2. Use of a cultured three-dimensional tissue comprising living fibroblasts in the manufacture of a medicament for treating a patient with congestive heart failure.

3. The use or cultured three-dimensional tissue for use according to any preceding claim, wherein said cultured three-dimensional tissue comprises a three-dimensional framework composed of biodegradable non-living material.

4. The use or cultured three-dimensional tissue for use according to claim 3, wherein said biodegradable material is selected from among the group consisting of: PLGA, TMC, PGA, PLA, polycaprolactone, catgut suture material, collagen, fibronectin, cellulose, gelatin, dextran, and hyaluronic acid.

5. The use or cultured three-dimensional tissue for use according to any preceding claim, wherein said cultured three-dimensional tissue further comprises a non-biodegradable material.

6. The use or cultured three-dimensional tissue for use according to Claim 1 or 2, wherein said cultured three-dimensional tissue comprises a three-dimensional framework composed of a hydrogel.

7. The use or cultured three-dimensional tissue for use according to any preceding claim, wherein said cultured three-dimensional tissue further comprises a living cell selected from among the group consisting of: stromal cell, smooth muscle cell, vascular smooth muscle cell, cardiac muscle cell, skeletal muscle cell, endothelial cell, pericyte, macrophage, monocyte, leukocyte, plasma cell, mast cell, adipocyte, stem cell, and progenitor cell.

8. The use or cultured three-dimensional tissue for use according to any preceding claim, wherein said cultured three-dimensional tissue is intended to be contacted with the patient's heart in a region selected from among the group consisting of: damaged or diseased heart tissue, epicardium, myocardium, and endocardium.

9. The use or cultured three-dimensional tissue for use according to any preceding claim, wherein said cultured three-dimensional tissue is capable of increasing heart ejection fraction by about 3% to 5%, about 5% to 10%, or more than 10%.

10. The use or cultured three-dimensional tissue for use according to any preceding claim, wherein said cultured three-dimensional tissue is capable of improving the pumping efficiency of the heart.

11. The use or cultured three-dimensional tissue for use according to any preceding claim, wherein said cultured three-dimensional tissue is to be applied in form of more than one cohesive piece of cultured three-dimensional tissue comprising living fibroblasts.

12. The use or cultured three-dimensional tissue for use according to any preceding claim, wherein said cultured three-dimensional tissue comprises interstitial spaces sized to allow living cells to stretch across them.

13. The use or cultured three-dimensional tissue for use according to claim 12, wherein said interstitial spaces are sized at least about 140 µm, 150 µm, 160 µm, 175 µm, 185 µm, 200 µm, 210 µm, or 220 µm,

14. The use or cultured three-dimensional tissue for use according to any preceding claim, wherein said cultured three-dimensional tissue is intended to be attached to the patient's heart with cellular attachment, biological glue, synthetic glue, laser dye, sutures, or hydrogel.

15. The use or cultured three-dimensional tissue for use according to any preceding claim, wherein said cultured three-dimensional tissue ranges in size from about 15 cm² to about 50 cm².

## Patentansprüche

1. Kultiviertes dreidimensionales Gewebe mit lebenden Fibroblasten zur Verwendung bei der Behandlung von kongestiver Herzinsuffizienz bei einem Patienten.

2. Verwendung eines kultivierten dreidimensionalen Gewebes mit lebenden Fibroblasten bei der Herstellung eines Medikaments für die Behandlung eines Patienten mit kongestiver Herzinsuffizienz.

3. Verwendung oder kultiviertes dreidimensionales Gewebe zur Verwendung nach einem der vorstehenden Ansprüche, wobei das kultivierte dreidimensionale Gewebe ein dreidimensionales Gerüst aufweist, das aus biologisch abbaubarem unbelebtem Material besteht.

4. Verwendung oder kultiviertes dreidimensionales Gewebe zur Verwendung nach Anspruch 3, wobei das biologisch abbaubare Material aus der Gruppe ausgewählt ist, die aus PLGA, TMC, PGA, PLA, Polycaprolacton, Katgutnahtmaterial, Kollagen, Fibronectin, Cellulose, Gelatine, Dextran und Hyaluronsäure besteht.

5. Verwendung oder kultiviertes dreidimensionales Gewebe zur Verwendung nach einem der vorstehenden Ansprüche, wobei das kultivierte dreidimensionale Gewebe ferner ein nicht biologisch abbaubares Material aufweist.

6. Verwendung oder kultiviertes dreidimensionales Gewebe zur Verwendung nach Anspruch 1 oder 2, wobei das kultivierte dreidimensionale Gewebe ein dreidimensionales Gerüst aufweist, das aus einem Hydrogel besteht.

7. Verwendung oder kultiviertes dreidimensionales Gewebe zur Verwendung nach einem der vorstehenden Ansprüche, wobei das kultivierte dreidimensionale Gewebe ferner eine lebende Zelle aufweist, die aus der Gruppe ausgewählt ist, die aus Stromazellen, glatten Muskelzellen, glatten Gefäßmuskelzellen, Herzmuskelzellen, Skelettmuskelzellen, Endothelzellen, Perizyten, Makrophagen, Monozyten, Leukozyten, Plasmazellen, Mastzellen, Adipozyten, Stammzellen und Progenitorzellen besteht.

8. Verwendung oder kultiviertes dreidimensionales Gewebe zur Verwendung nach einem der vorstehenden Ansprüche, wobei das kultivierte dreidimensionale Gewebe mit dem Herz eines Patienten in einer Region in Kontakt gebracht werden soll, die aus der Gruppe ausgewählt ist, die aus geschädigtem oder erkranktem Herzgewebe, Epikardium, Myokardium und Endokardium besteht.

9. Verwendung oder kultiviertes dreidimensionales Gewebe zur Verwendung nach einem der vorstehenden Ansprüche, wobei das kultivierte dreidimensionale Gewebe imstande ist, die Herzauswurffraktion um etwa 3% bis 5%, etwa 5% bis 10% oder um mehr als 10% zu erhöhen.

10. Verwendung oder kultiviertes dreidimensionales Gewebe zur Verwendung nach einem der vorstehenden Ansprüche, wobei das kultivierte dreidimensionale Gewebe imstande ist, die Pumpleistung des Herzes zu verbessern.

11. Verwendung oder kultiviertes dreidimensionales Gewebe zur Verwendung nach einem der vorstehenden Ansprüche, wobei das kultivierte dreidimensionale Gewebe in Form von mehr als einem zusammenhängenden Stück von kultiviertem dreidimensionalem Gewebe mit lebenden Fibroblasten aufzubringen ist.

12. Verwendung oder kultiviertes dreidimensionales Gewebe zur Verwendung nach einem der vorstehenden Ansprüche, wobei das kultivierte dreidimensionale Gewebe Interstitialräume aufweist, die so bemessen sind, dass lebende Zellen sie überspannen können.

13. Verwendung oder kultiviertes dreidimensionales Gewebe zur Verwendung nach Anspruch 12. wobei die Größen der Interstitialräume mindestens etwa 140 µm, 150 µm, 160 µm, 175 µm, 185 µm, 200 µm, 210 µm oder 220 µm betragen.

14. Verwendung oder kultiviertes dreidimensionales Gewebe zur Verwendung nach einem der vorstehenden Ansprüche, wobei das kultivierte dreidimensionale Gewebe an dem Herz des Patienten durch zelluläre Bindung, biologischen Klebstoff, synthetischen Klebstoff Laserfarbstoff. Nähte oder Hydrogel befestigt werden soll.

15. Verwendung oder kultiviertes dreidimensionales Gewebe zur Verwendung nach einem der vorstehenden Ansprüche, wobei die Größe des kultivierten dreidimensionalen Gewebes im Bereich von etwa 15 cm² bis etwa 50 cm² liegt.

## Revendications

1. Tissu tridimensionnel cultivé comprenant des fibroblastes vivants pour utilisation dans le traitement de l'insuffisance cardiaque congestive chez un patient.

2. Utilisation d'un tissu tridimensionnel cultivé comprenant des fibroblastes vivants dans la fabrication d'un médicament pour traiter un patient ayant une insuffisance cardiaque congestive.

3. Utilisation ou tissu tridimensionnel cultivé pour utilisation selon l'une quelconque des revendications précédentes, où ledit tissu tridimensionnel cultivé comprend une matrice tridimensionnelle composée de matériau non vivant biodégradable.

4. Utilisation ou tissu tridimensionnel cultivé pour utilisation selon la revendication 3, où ledit matériau biodégradable est choisi dans le groupe constitué de :
PLGA. TMC, PGA, PLA, la polycaprolactone, un matériau de suture catgut, le collagène, la fibronectine, la cellulose, la gélatine, le dextrane, et l'acide hyaluronique.

5. Utilisation ou tissu tridimensionnel cultivé pour utilisation selon l'une quelconque des revendications précédentes, où ledit tissu tridimensionnel cultivé comprend en outre un matériau non biodégradable.

6. Utilisation ou tissu tridimensionnel cultivé pour utilisation selon la revendication 1 ou 2, dans lequel ledit tissu tridimensionnel cultivé comprend une matrice tridimensionnelle composée d'un hydrogel.

7. Utilisation ou tissu tridimensionnel cultivé pour utilisation selon l'une quelconque des revendications précédentes, où ledit tissu tridimensionnel cultivé comprend en outre une cellule vivante choisie dans le groupe constitué de : une cellule stromale, une cellule de muscle lisse, une cellule de muscle lisse vasculaire, une cellule de muscle cardiaque, une cellule de muscle squelettique, une cellule endothéliale, un péricyte, un macrophage, un monocyte, un leucocyte, un plasmocyte, un mastocyte, un adipocyte, une cellule souche, et une cellule progénitrice.

8. Utilisation ou tissu tridimensionnel cultivé pour utilisation selon l'une quelconque des revendications précédentes, où ledit tissu tridimensionnel cultivé est destiné à être en contact avec le coeur du patient dans une région choisie dans le groupe constitué de : un tissu cardiaque endommagé ou malade, l'épicarde, le myocarde, et l'endocarde.

9. Utilisation ou tissu tridimensionnel cultivé pour utilisation selon l'une quelconque des revendications précédentes, où ledit tissu tridimensionnel cultivé est capable d'augmenter la fraction d'éjection cardiaque d'environ 3 % à 5 %, d'environ 5 % à 10 %, ou plus de 10 %.

10. Utilisation ou tissu tridimensionnel cultivé pour utilisation selon l'une quelconque des revendications précédentes, où ledit tissu tridimensionnel cultivé est capable d'améliorer l'efficacité de pompage du coeur.

11. Utilisation ou tissu tridimensionnel cultivé pour utilisation selon l'une quelconque des revendications précédentes, où ledit tissu tridimensionnel cultivé est destiné à être appliqué sous la forme de plus d'une pièce cohésive de tissu tridimensionnel cultivé comprenant des fibroblastes vivants.

12. Utilisation ou tissu tridimensionnel cultivé pour utilisation selon l'une quelconque des revendications précédentes, où ledit tissu tridimensionnel cultivé comprend des espaces interstitiels dimensionnés de manière à permettre à des cellules vivantes de s'étendre dans ceux-ci.

13. Utilisation ou tissu tridimensionnel cultivé pour utilisation selon la revendication 12, où lesdits espaces interstitiels sont dimensionnés à au moins environ 140 µm, 150 µm, 160 µm, 175 µm, 185 µm, 200 µm, 210 µm, ou 220 µm.

14. Utilisation ou tissu tridimensionnel cultivé pour utilisation selon l'une quelconque des revendications précédentes, où ledit tissu tridimensionnel cultivé est destiné à être fixé au coeur du patient avec une fixation cellulaire, une colle biologique, une colle synthétique, un colorant laser, des sutures, ou un hydrogel.

15. Utilisation ou tissu tridimensionnel cultivé pour utilisation selon l'une quelconque des revendications précédentes, dans lequel ledit tissu tridimensionnel cultivé a une taille dans la plage d'environ 15 cm² à environ 50 cm².
